# EUROPÄISCHE PATENTANMELDUNG

(11) **EP 0 779 279 A1**
(43) Veröffentlichungstag der Anmeldung: **18.06.1997**
(21) Anmeldenummer: 96119321.6
(22) Anmeldetag: 03.12.1996
(51) Int. Cl.: C07D 239/90, C07D 235/08, C07D 235/28, C07D 417/04, C07D 409/04, C07D 401/04, C07D 263/58, C07D 265/36, A61K 31/415, A61K 31/505

(54) **Bicyclische Heterocyclen**

(30) Priorität: 15.12.1995 DE 19546918
(71) Anmelder: BAYER AG, 51368 Leverkusen (DE)
(72) Erfinder: Connell, Richard, Dr., Trumbull, CT 06611 (US); Goldmann, Siegfried, Dr., 42327 Wuppertal (DE); Müller, Ulrich, Dr., 42111 Wuppertal (DE); Lohmer, Stefan, Dr., 20133 Milano (IT); Bischoff, Hilmar, Dr., 42113 Wuppertal (DE); Denzer, Dirk, Dr., 42115 Wuppertal (DE); Grützmann, Rudi, Dr., 42657 Solingen (DE); Wohlfeil, Stefan, Dr., 40721 Hilden (DE)

(57) **Zusammenfassung**

Die bicyclischen Heterocyclen werden hergestellt durch Umsetzung von entsprechend substituierten Carbonsäuren mit Aminen, insbesondere mit Phenylglycinolamin. Die erfindungsgemäßen bicyclischen Heterocyclen eignen sich als Wirkstoffe in Arzneimitteln, insbesondere in Arzneimittel mit antiatherosklerotischer Wirkung.

## Beschreibung

Die vorliegende Erfindung betrifft bicyclische Heterocyclen, Verfahren zu ihrer Herstellung und ihre Verwendung in Arzneimitteln, insbesondere als antiatherosklerotische Arzneimittel.

Es ist bekannt, daß erhöhte Blutspiegel von Triglyceriden (Hypertriglyceridämie) und Cholesterin (Hypercholesterinämie) mit der Genese von atherosklerotischen Gefäßwand-Veränderungen und koronaren Herzkrankheiten assoziiert sind.

Ein deutlich erhöhtes Risiko für die Entwicklung koronarer Herzerkrankungen liegt darüberhinaus vor, wenn diese beiden Risikofaktoren kombiniert auftreten, was wiederum mit einer Überproduktion an Apolipoprotein B-100 einhergeht. Es ist daher nach wie vor ein starkes Bedürfnis, wirksame Arzneimittel zur Bekämpfung der Atherosklerose sowie koronarer Herzkrankheiten zur Verfügung zu stellen.

Außerdem werden in dem US-Patent 5 314 880 Benzimidazolderivate mit einer PAF-antagonistischen Wirkung beschrieben.

Die vorliegende Erfindung betrifft bicyclische Heterocyclen der allgemeinen Formel (I) 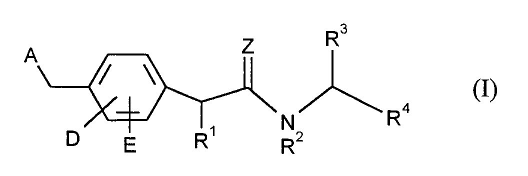 in welcher
- A: für einen Rest der Formel 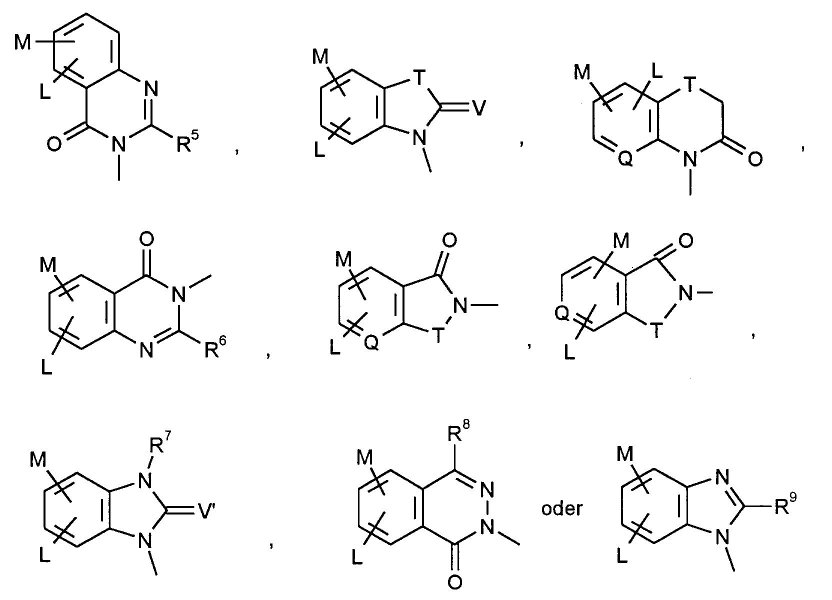 steht,
worin
- L und M: gleich oder verschieden sind und
Wasserstoff, Halogen, Trifluormethyl, Carboxyl, Cycloalkyl mit 3 bis 6 Kohlenstoffatomen, Hydroxy, Phenyl oder geradkettiges oder verzweigtes Alkyl, Alkoxycarbonyl oder Alkoxy mit jeweils bis zu 6 Kohlenstoffatomen bedeuten,
- Q: ein Stickstoffatom oder die -CH-Gruppe bedeutet,
- T: eine Gruppe der Formel -SO₂ oder -CO oder
ein Sauerstoff- oder Schwefelatom bedeutet,
- V: ein Sauerstoff- oder Schwefelatom bedeutet,
- R⁵, R⁶, R⁷ und R⁸: gleich oder verschieden sind und
Wasserstoff, geradkettiges oder verzweigtes Alkyl mit bis zu 6 Kohlenstoffatomen, Benzyl oder Phenyl bedeuten, die gegebenenfalls durch Halogen oder durch geradkettiges oder verzweigtes Alkyl mit bis zu 6 Kohlenstoffatomen substituiert sind,
- R⁹: Trifluormethyl, Benzyl oder einen 5- bis 7-gliedrigen, gegebenenfalls benzokondensierten Heterocyclus mit bis zu 3 Heteratomen aus der Reihe S, N und/oder O bedeutet, der gegebenenfalls bis zu 3-fach gleich oder verschieden durch Halogen, Phenyl, Hydroxy oder durch geradkettiges oder verzweigtes Alkyl oder Alkoxy mit jeweils bis zu 4 Kohlenstoffatomen substituiert ist, oder eine Gruppe der Formel -S(O)ₐ-R¹⁰ bedeutet,
worin
- a: eine Zahl 0, 1 oder 2 bedeutet,
- R¹⁰: geradkettiges oder verzweigtes Alkyl oder Alkenyl mit jeweils bis zu 8 Kohlenstoffatomen bedeutet, die gegebenenfalls durch geradkettiges oder verzweigtes Acyl mit bis zu 6 Kohlenstoffatomen oder durch Aryl oder Aroyl mit jeweils bis zu 10 Kohlenstoffatomen substituiert sind, die ihrerseits bis zu 2-fach gleich oder verschieden durch Halogen, Trifluormethyl oder durch geradkettiges oder verzweigtes Acyl mit bis zu 5 Kohlenstoffatomen substituiert sein können, oder
Aryl mit 6 bis 10 Kohlenstoffatomen bedeutet, die gegebenenfalls durch Halogen, Hydroxy, Trifluormethyl oder geradkettiges oder verzweigtes Alkyl oder Alkoxy mit jeweils bis zu 5 Kohlenstoffatomen substituiert ist,
- D und E: gleich oder verschieden sind und
für Wasserstoff Halogen, Trifluormethyl, Hydroxy, Carboxyl oder für geradkettiges oder verzweigtes Alkyl, Alkoxy oder Alkoxycarbonyl mit jeweils bis zu 6 Kohlenstoffatomen stehen,
- Z: für ein Sauerstoff- oder Schwefelatom steht,
- R¹: für Cycloalkyl mit 3 bis 10 Kohlenstoffatomen oder für geradkettiges oder verzweigtes Alkyl mit 1 bis 10 Kohlenstoffatomen steht, oder
für Phenyl steht, das gegebenenfalls bis zu 2-fach gleich oder verschieden durch Halogen, Nitro, Cyano, Hydroxy, geradkettiges oder verzweigtes Alkyl oder Alkoxy mit jeweils bis zu 4 Kohlenstoffatomen substituiert ist,
- R²: für Wasserstoff oder geradkettiges oder verzweigtes Alkyl mit bis zu 3 Kohlenstoffatomen steht,
- R³: für Wasserstoff oder geradkettiges oder verzweigtes Alkyl mit bis zu 5 Kohlenstoffatomen steht, oder
für Cycloalkyl mit 3 bis 7 Kohlenstoffatomen steht, oder
für Phenyl oder für einen 5- bis 7-gliedrigen aromatischen Heterocyclus mit bis zu 3 Heteroatomen aus der Reihe S, N und/oder O steht, die gegebenenfalls bis zu 3-fach gleich oder verschieden durch Halogen, Nitro, Phenyl, Hydroxy oder durch geradkettiges oder verzweigtes Alkyl oder Alkoxy mit bis zu 6 Kohlenstoffatomen substituiert sind,
- R⁴: für Wasserstoff oder für eine Gruppe der Formel -CH₂-OH oder CH₂O-CO-R¹¹ steht,
worin
- R¹¹: Wasserstoff, geradkettiges oder verzweigtes Alkyl mit bis zu 8 Kohlenstoffatomen oder Phenyl bedeutet, das gegebenenfalls bis zu 3-fach gleich oder verschieden durch Halogen, Hydroxy, Cyano oder geradkettiges oder verzweigtes Alkyl oder Alkoxy mit jeweils bis zu 4 Kohlenstoffatomen substituiert ist,
und deren Salze.

Die erfindungsgemäßen bicyclischen Heterocyclen können auch in Form ihrer Salze vorliegen. Im allgemeinen seien hier Salze mit organischen oder anorganischen Basen oder Säuren genannt.

Im Rahmen der vorliegenden Erfindung werden physiologisch unbedenkliche Salze bevorzugt. Physiologisch unbedenkliche Salze der erfindungsgemäßen Verbindungen können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Physiologisch unbedenkliche Salze können ebenso Metall- oder Ammoniumsalze der erfindungsgemäßen Verbindungen sein, welche eine freie Carboxylgruppe besitzen, sein. Besonders bevorzugt sind z.B. Natrium-, Kalium-, Magnesium- oder Calciumsalze, sowie Ammoniumsalze, die abgeleitet sind von Ammoniak, oder organischen Aminen, wie beispielsweise Ethylamin, Di-bzw. Triethylamin, Di- bzw. Triethanolamin, Dicyclohexylamin, Dimethylaminoethanol, Arginin, Lysin, Ethylendiamin oder 2-Phenylethylamin.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren oder deren jeweiligen Mischungen. Diese Mischungen der Enantiomeren und Diastereomeren lassen sich in bekannter Weise in die stereoisomer einheitlichen Bestandteile trennen.

Heterocyclus, gegebenenfalls benzokondensiert, steht im Rahmen der Erfindung im allgemeinen für einen gesättigten oder ungesättigten 5- bis 7-gliedrigen, vorzugsweise 5- bis 6-gliedrigen Heterocyclus der bis zu 3 Heteroatome aus der Reihe S, N und/oder O enthalten kann und der im Fall eines Stickstoffatoms auch über dieses gebunden sein kann. Beispielsweise seien genannt: Indolyl, Chinolyl, Benzo[b]thienyl, Benzo[b]furyl, Pyridyl, Thienyl, Furyl, Pyrrolyl, Thiazolyl, Oxazolyl, Imidazolyl, Morpholinyl oder Piperidyl. Bevorzugt sind Chinolyl, Furyl, Pyridyl und Thienyl.

Bevorzugt sind Verbindungen der allgemeinen Formel (I),
in welcher
- A: für einen Rest der Formel 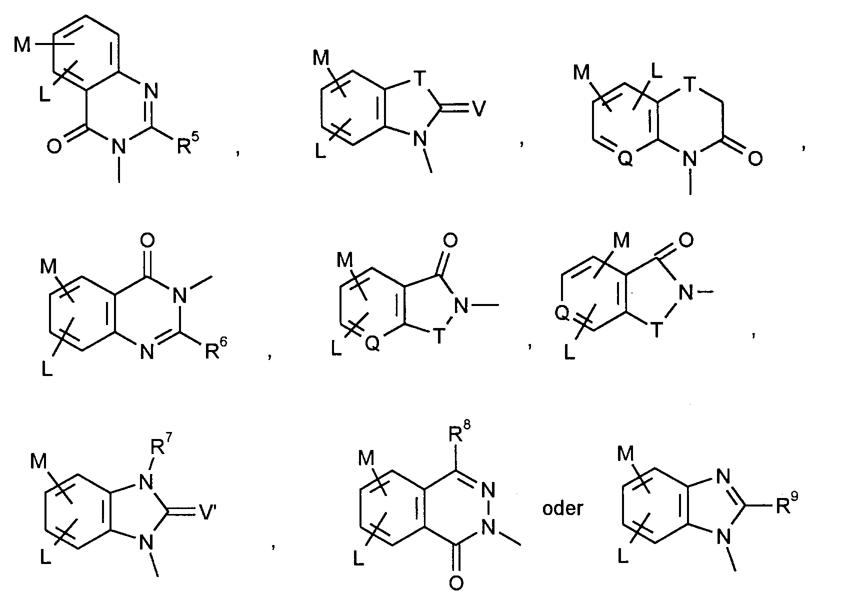 steht,
worin
- L und M: gleich oder verschieden sind und
Wasserstoff Fluor, Chlor, Brom, Trifluormethyl, Cyclopropyl, Cyclobutyl, Cyclopentyl, Cyclohexyl, Hydroxy, Phenyl oder geradkettiges oder verzweigtes Alkyl, Alkoxycarbonyl oder Alkoxy mit jeweils bis zu 5 Kohlenstoffatomen bedeuten,
- Q: ein Stickstoffatom oder die -CH-Gruppe bedeutet,
- T: eine Gruppe der Formel -SO₂ oder -CO oder
ein Sauerstoff- oder Schwefelatom bedeutet,
- V: ein Sauerstoff- oder Schwefelatom bedeutet,
- R⁵, R⁶, R⁷ und R⁸: gleich oder verschieden sind und
Wasserstoff geradkettiges oder verzweigtes Alkyl mit bis zu 5 Kohlenstoffatomen, Benzyl oder Phenyl bedeuten, die gegebenenfalls durch Fluor, Chlor, Brom oder durch geradkettiges oder verzweigtes Alkyl mit bis zu 5 Kohlenstoffatomen substituiert sind,
- R⁹: Trifluormethyl, Benzyl, Benzothienyl, Thienyl, Pyridyl, Chinolyl, Imidazolyl, Furyl, Pyrryl, Oxazolyl oder Thiazolyl bedeutet, die gegebenenfalls bis zu 3-fach gleich oder verschieden durch Fluor, Chlor, Brom, Phenyl, Hydroxy oder durch geradkettiges oder verzweigtes Alkyl oder Alkoxy mit jeweils bis zu 3 Kohlenstoffatomen substituiert sind, oder
eine Gruppe der Formel -S(O)ₐ-R¹⁰ bedeutet,
worin
- a: eine Zahl 0 oder 1 bedeutet,
- R¹⁰: geradkettiges oder verzweigtes Alkyl oder Alkenyl mit jeweils bis zu 6 Kohlenstoffatomen bedeutet, die gegebenenfalls durch geradkettiges oder verzweigtes Acyl mit bis zu 5 Kohlenstoffatomen oder durch Phenyl, Benzoyl oder Naphthyl substituiert sind, die ihrerseits bis zu 2-fach gleich oder verschieden durch Fluor, Chlor, Brom, Trifluormethyl oder durch geradkettiges oder verzweigtes Acyl mit bis zu 4 Kohlenstoffatomen substituiert sein können, oder
Naphthyl oder Phenyl bedeutet, die gegebenenfalls durch Fluor, Chlor, Brom, Hydroxy, Trifluormethyl oder geradkettiges oder verzweigtes Alkyl oder Alkoxy mit jeweils bis zu 4 Kohlenstoffatomen substituiert sind,
- D und E: gleich oder verschieden sind und
für Wasserstoff, Fluor, Chlor, Brom, Trifluormethyl, Hydroxy oder geradkettiges oder verzweigtes Alkyl oder Alkoxy mit jeweils bis zu 4 Kohlenstoffatomen stehen,
- Z: für ein Sauerstoff- oder Schwefelatom steht,
- R¹: für Cyclobutyl, Cyclopentyl, Cyclohexyl, Cycloheptyl, Cylcooctyl oder für geradkettiges oder verzweigtes Alkyl mit bis zu 7 Kohlenstoffatomen steht, oder
für Phenyl steht, das gegebenenfalls durch Fluor, Chlor, Brom, Nitro, Cyano, Hydroxy oder geradkettiges oder verzweigtes Alkyl oder Alkoxy mit jeweils bis zu 3 Kohlenstoffatomen substituiert ist,
- R²: Wasserstoff oder Methyl bedeutet,
- R³: für Wasserstoff oder geradkettiges oder verzweigtes Alkyl mit bis zu 4 Kohlenstoffatomen, Benzyl, Cyclopropyl, Cyclopentyl, Cyclohexyl oder für Phenyl, Pyridyl, Thienyl oder Furyl steht, die gegebenenfalls bis zu 2-fach gleich oder verschieden durch Fluor, Chlor, Brom, Phenyl, Nitro, Hydroxy oder durch geradkettiges oder verzweigtes Alkyl oder Alkoxy mit bis zu 4 Kohlenstoffatomen substituiert sind,
- R⁴: für Wasserstoff oder für eine Gruppe der Formel -CH₂-OH oder -CH₂O-CO-R¹¹ steht,
worin
- R¹¹: Wasserstoff, geradkettiges oder verzweigtes Alkyl mit bis zu 6 Kohlenstoffatomen oder Phenyl bedeutete, das gegebenenfalls bis zu 2-fach gleich oder verschieden durch Fluor, Chlor, Brom, Cyano, Hydroxy oder geradkettiges oder verzweigtes Alkyl oder Alkoxy mit jeweils bis zu 3 Kohlenstoffatomen substituiert ist,
und deren Salze.

Besonders bevorzugt sind Verbindungen der allgemeinen Formel (I),
in welcher
- A: für einen Rest der Formel 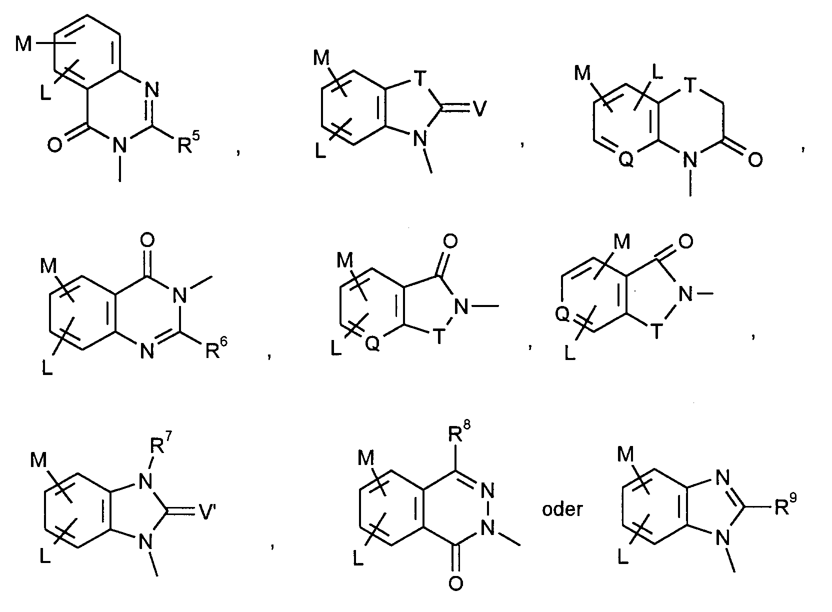 steht,
worin
- L und M: gleich oder verschieden sind und
Wasserstoff, Fluor, Chlor, Brom, Hydroxy, Phenyl oder geradkettiges oder verzweigtes Alkyl oder Alkoxy mit jeweils bis zu 4 Kohlenstoffatomen bedeuten,
- Q: ein Stickstoffatom oder die -CH-Gruppe bedeutet,
- T: eine Gruppe der Formel -SO₂ oder -CO oder
ein Sauerstoff- oder Schwefelatom bedeutet,
- V: ein Sauerstoff- oder Schwefelatom bedeutet,
- R⁵, R⁶, R⁷ und R⁸: gleich oder verschieden sind und
Wasserstoff geradkettiges oder verzweigtes Alkyl mit bis zu 4 Kohlenstoffatomen, Benzyl oder Phenyl bedeuten, die gegebenenfalls durch Fluor, Chlor, Brom oder durch geradkettiges oder verzweigtes Alkyl mit bis zu 4 Kohlenstoffatomen substituiert sind,
- R⁹: Trifluormethyl, Benzyl, Benzothienyl, Thienyl, Pyridyl, Imidazolyl, Furyl oder Thiazolyl bedeutet, die gegebenenfalls bis zu 3-fach gleich oder verschieden durch Fluor, Chlor, Brom, Phenyl, Hydroxy oder durch geradkettiges oder verzweigtes Alkyl oder Alkoxy mit jeweils bis zu 3 Kohlenstoffatomen substituiert sind, oder
eine Gruppe der Formel -S(O)ₐ-R¹⁰ bedeutet,
worin
- a: eine Zahl 0 oder 1 bedeutet,
- R¹⁰: geradkettiges oder verzweigtes Alkyl oder Alkenyl mit jeweils bis zu 5 Kohlenstoffatomen bedeutet, die gegebenenfalls durch geradkettiges oder verzweigtes Acyl mit bis zu 4 Kohlenstoffatomen oder durch Phenyl, Benzoyl oder Naphthyl substituiert sind, die ihrerseits bis zu 2-fach gleich oder verschieden durch Fluor, Chlor, Brom, Trifluormethyl oder durch geradkettiges oder verzweigtes Acyl mit bis zu 3 Kohlenstoffatomen substituiert sein können, oder
Naphthyl oder Phenyl bedeutet, die gegebenenfalls durch Fluor, Chlor, Brom, Hydroxy, Trifluormethyl oder geradkettiges oder verzweigtes Alkyl oder Alkoxy mit jeweils bis zu 3 Kohlenstoffatomen substituiert sind,
- D und E: gleich oder verschieden sind und
für Wasserstoff, Fluor, Chlor, Brom oder Trifluormethyl stehen,
- Z: für Sauerstoff steht,
- R¹: für Cyclopentyl, Cyclohexyl, Cycloheptyl, Cylcooctyl oder für geradkettiges oder verzweigtes Alkyl mit bis zu 6 Kohlenstoffatomen steht,
- R²: für Wasserstoff oder Methyl steht,
- R³: für Phenyl steht und
- R⁴: für die Gruppe -CH₂-OH steht,
und deren Salze.

Außerdem wurde ein Verfahren zur Herstellung der erfindungsgemäßen Verbindungen der allgemeinen Formel (I) gefunden,
dadurch gekennzeichnet, daß man
Säuren der allgemeinen Formel (II) 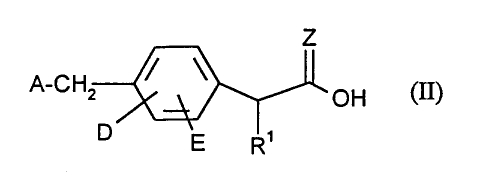 in welcher
A, D, E, Z und R¹ die angegebene Bedeutung haben,
mit Verbindungen der allgemeinen Formel (III) 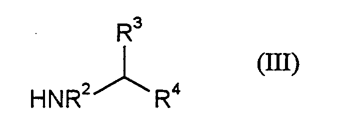 in welcher
R², R³ und R⁴ die angegebene Bedeutung haben,
in inerten Lösemitteln und in Anwesenheit von Basen und/oder Hilfsstoffen umsetzt.

Das erfindungsgemäße Verfahren kann durch folgendes Formelschema beispielhaft erläutert werden:

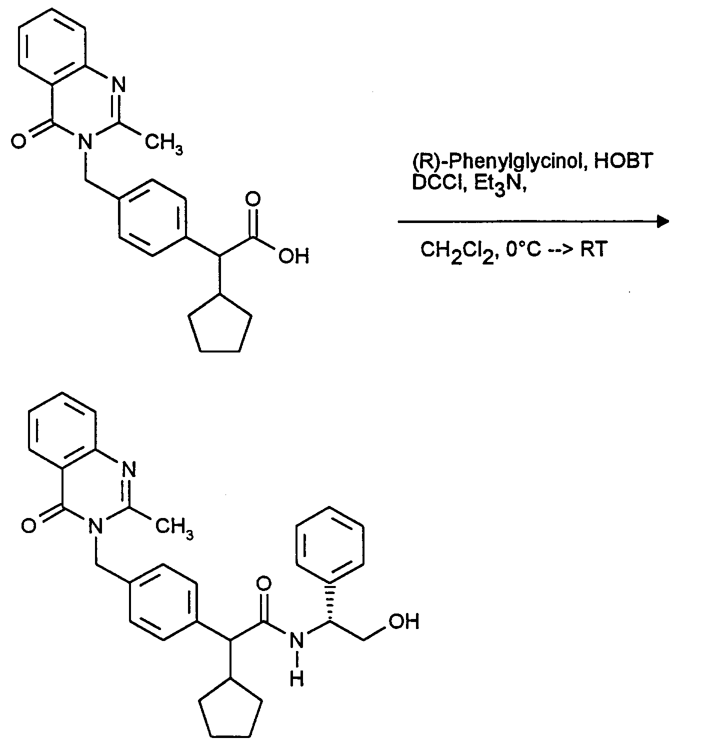

Als Lösemittel eignen sich hierbei inerte organische Lösemittel, die sich unter den Reaktionsbedingungen nicht verändern. Hierzu gehören Ether, wie Diethylether oder Tetrahydrofuran, Halogenkohlenwasserstoffe wie Dichlormethan, Trichlormethan, Tetrachlormethan, 1,2-Dichlorethan, Trichlorethan, Tetrachlorethan, 1,2-Dichlorethan oder Trichlorethylen, Kohenwasserstoffe wie Benzol, Xylol, Toluol, Hexan, Cyclohexan, oder Erdölfraktionen, Nitromethan, Dimethylformamid, Aceton, Acetonitril oder Hexamethylphosphorsäuretriamid. Ebenso ist es möglich, Gemische der Lösemittel einzusetzen. Besonders bevorzugt sind Dichlormethan, Tetrahydrofuran, Toluol oder Dimethylformamid.

Als Basen für das erfindungsgemäße Verfahren können im allgemeinen anorganische oder organische Basen eingesetzt werden. Hierzu gehören vorzugsweise Alkalihydroxide wie zum Beispiel Natriumhydroxid oder Kaliumhydroxid, Erdalkalihydroxide wie zum Beispiel Bariumhydroxid, Alkalicarbonate wie Natriumcarbonat oder Kaliumcarbonat, Erdalkalicarbonate wie Calciumcarbonat, oder Alkali- oder Erdalkalialkoholate wie Natrium- oder Kaliummethanolat, Natrium- oder Kaliumethanolat oder Kalium-tert.butylat, oder organische Amine (Trialkyl(C₁-C₆)amine) wie Triethylamin, oder Heterocyclen wie 1,4-Diazabicyclo[2.2.2]octan (DABCO), 1,8-Diazabicyclo[5.4.0]undec-7-en (DBU), Pyridin, Diaminopyridin, Methylpiperidin oder Morpholin. Es ist auch möglich als Basen Alkalimetalle wie Natrium und deren Hydride wie Natriumhydrid einzusetzen. Bevorzugt sind Natrium- und Kaliumcarbonat und Triethylamin.

Die Base wird in einer Menge von 1 mol bis 5 mol, bevorzugt von 1 mol bis 3 mol, bezogen auf 1 mol der Verbindung der allgemeinen Formel (II) eingesetzt.

Als Hilfsmittel eignen sich ebenso Dehydratisierungsreagenzien. Dazu gehören beispielsweise Carbodiimide wie Diisopropylcarbodiimid, Dicyclohexylcarbodiimid oder N-(3-Dimethylaminopropyl)-N'-ethylcarbodiimid-Hydrochlorid oder Carbonylverbindungen wie Carbonyldiimidazol oder 1,2-Oxazoliumverbindungen wie 2-Ethyl-5-phenyl-1,2-oxazolium-3-sulfonat oder Propanphosphorsäureanhydrid oder Iso-butylchloroformat oder Benzotriazolyloxy-tris-(dimethylamino)phosphoniumhexa-fluorophosphat oder Phosphonsäurediphenyl-esteramid oder Methan-sulfonsäurechlorid, gegebenenfalls in Anwesenheit von Basen wie Triethylamin oder N-Ethylmorpholin oder N-Methylpiperidin oder Dicyclohexylcarbodiimid und N-Hydroxysuccinimid.

Die Reaktion wird im allgemeinen in einem Temperaturbereich von 0°C bis 150°C, bevorzugt von +20°C bis +110°C durchgeführt.

Die Umsetzung kann bei normalen, erhöhtem oder bei erniedrigtem Druck durchgeführt werden (z.B. 0,5 bis 5 bar). Im allgemeinen arbeitet man bei Normaldruck.

Die Verbindungen der allgemeinen Formel (II) sind größtenteils neu und können beispielsweise hergestellt werden, indem man
Verbindungen der allgemeinen Formel (IV)

A-H (IV)

in welcher
- A: die angegebene Bedeutung hat, mit Verbindugen der allgemeinen Formel (V) 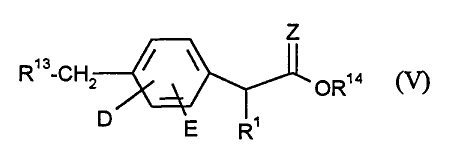 in welcher
- D, E, Z, R¹: die angegebene Bedeutung haben,
- R¹³: für Halogen, vorzugsweise für Chlor oder Brom steht,
und
- R¹⁴: für geradkettiges oder verzweigtes Alkyl mit bis zu 6 Kohlenstoffatomen steht,
in inerten Lösemitteln und in Anwesenheit von Basen und/oder Hilfsmitteln umsetzt,
und abschließend die Ester nach üblichen Methoden hydrolysiert.

Für die Umsetzung eignen sich übliche organische Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, Triethylamin, Pyridin, Dimethylsulfoxid, Dimethylformamid, Hexamethylphosphorsäuretriamid, Acetonitril, Aceton oder Nitromethan. Ebenso ist es möglich, Gemische der genannten Lösemittel zu verwenden. Bevorzugt sind Dimethylformamid, Tetrahydrofuran und Dimethylsulfoxid.

Als Basen können im allgemeinen die oben aufgeführten Basen, insbesondere aber die Alkalimetallhydride wie Natriumhydrid eingesetzt werden.

Im allgemeinen setzt man die Base in einer Menge von 0,05 mol bis 10 mol, bevorzugt von 1 mol bis 2 mol, bezogen auf 1 mol der Verbindung der Formel (IV) ein.

Der Hilfsstoff wird im allgemeinen in einer Menge von 0.01 mol bis 100 mol, bevorzugt von 0.1 mol bis 10 mol, bezogen auf 1 mol der Verbindung der allgemeinen Formel (IV) eingesetzt.

Die Umsetzung wird im allgemeinen in einem Temperaturbereich von -30°C bis +100°C, bevorzugt von -10°C bis +60°C, durchgeführt.

Die Umsetzung wird im allgemeinen bei Normaldruck durchgeführt. Es ist aber auch möglich, die Verfahren bei Überdruck oder bei Unterdruck durchzuführen (z.B. in einem Bereich von 0,5 bis 5 bar).

Als Hilfsmittel eignen sich die oben aufgeführten Dehydratisierungsreagenzien.

Die Herstellung der verschiedenen Amin/Amidderivate erfolgt auch unter Anwendung einer Vielzahl von bekannten Methoden, wie beispielsweise die Kopplung von Säurederivaten aus der Peptidchemie [vgl. hierzu Bodanszky, The Practice of Peptide Synthesis: Springer Verlag, Vol 21, 1984 and Organic Chemistry; Functional Group Transformations: Academic Press, Vol 12-1].

Die Esterhydrolyse erfolgt nach üblichen Methoden, indem man die Ester in inerten Lösemitteln mit üblichen Basen behandelt.

Als Basen eignen sich die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat. Besonders bevorzugt werden Natriumhydroxid oder Kaliumhydroxid eingesetzt.

Als Lösemittel eignen sich Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Die Esterhydrolyse wird im allgemeinen in einem Temperaturbereich von 0°C bis +100°C, bevorzugt von +20°C bis +80°C durchgeführt.

Im allgemeinen wird die Esterhydrolyse bei Normaldruck durchgeführt. Es ist aber auch möglich, bei Unterdruck oder bei Überdruck zu arbeiten (z.B. von 0,5 bis 5 bar).

Bei der Durchführung der Esterhydrolyse wird die Base im allgemeinen in einer Menge von 1 bis 3 Mol, bevorzugt von 1 bis 1,5 Mol bezogen auf 1 Mol des Esters eingesetzt. Besonders bevorzugt verwendet man molare Mengen der Reaktanden.

Die Verbindungen der allgemeinen Formel (III) sind an sich bekannt.

Die erfindungsgemäßen Verbindungen der allgemeinen Formel (I) haben ein nicht vorhersehbares pharmakologisches Wirkspektrum.

Sie können als Wirkstoffe in Arzneimitteln zur Reduzierung von Veränderungen an Gefäßwänden Verwendung finden und zur Behandlung von Koronaren Herzerkrankungen, Herzinsuffizienz, Störungen der Hirnleistung, ischämischen Gehirnerkrankungen, Apoplex, Durchblutungsstörungen, Mikrozirkulationsstörungen und Thrombosen.

Weiterhin spielt bei der Okklusion von Gefäßen die Proliferation glatter Muskelzellen eine ausschlaggebende Rolle. Die erfindungsgemäßen Verbindungen sind geeignet, diese Proliferation zu inhibieren und damit atherosklerotische Prozesse zu verhindern.

Die erfindungsgemäßen Verbindungen zeichnen sich durch eine Senkung der ApoB-100-assoziierten Lipoproteinen (VLDL und seiner Abbauprodukte, wie z.B. LDL), des ApoB-100, der Triglyceride und des Cholesterins aus. Damit besitzen sie wertvolle, im Vergleich zum Stand der Technik überlegene pharmakologische Eigenschaften.

Überraschenderweise besteht die Wirkung der erfindungsgemäßen Verbindungen zunächst in einer Verminderung oder vollständigen Inhibierung der Bildung und/oder der Freisetzung von ApoB-100-assoziierten Lipoproteinen aus Leberzellen, was eine Senkung des VLDL-Plasmaspiegels zur Folge hat. Diese VLDL-Senkung muß mit einer Senkung der Plasmaspiegel von ApoB-100, LDL, Triglyceriden und von Cholesterin einhergehen; es werden also gleichzeitig mehrere der obengenannten Risikofaktoren gesenkt, die an Gefäßwandveränderungen beteiligt sind.

Die erfindungsgemäßen Verbindungen können daher zur Präventation und Behandlung von Atherosklerose, der Fettsucht, Pankreatitis und der Obstipation eingesetzt werden.

### 1. Hemmung der Freisetzung ApoB-100-assoziierter Lipoproteine

Der Test zum Nachweis der Hemmung der Freisetzung ApoB-100-assoziierter Liproproteine aus Leberzellen erfolgte in vitro mit kultivierten Leberzellen, bevorzugt mit Zellen der humanen Linie HepG2. Diese Zellen werden unter Standardbedingungen in Medium für die Kultur eukariontischer Zellen gezüchtet, bevorzugt in RPMI 1640 mit 10% fötalem Kälberserum. HepG2-Zellen synthetisieren und sezernieren in den Kulturüberstand ApoB-100-assoziierte Lipoproteinpartikel, die im Prinzip ähnlich aufgebaut sind wie die VLDL- bzw. LDL-Partikel, die im Plasma zu finden sind.

Diese Partikel können mit einem Immunoassay für humanes LDL nachgewiesen werden. Dieser Immunoassay erfolgt mit Antikörpern, die im Kaninchen gegen humanes LDL unter Standardbedingungen induziert worden waren. Die anti-LDL-Antikörper (Kan-anti-LDL-Ak) wurden an einem Immunosorbens mit humanem LDL affinitätschromatographisch gereinigt. Diese gereinigten Kan-anti-LDL-Ak werden an die Oberfläche von Plastik adsorbiert. Zweckmäßigerweise erfolgt diese Adsorbtion an die Plastikoberfläche von Mikrotitierplatten mit 96 Vertiefungen, bevorzugt an MaxiSorp-Platten. Wenn im Überstand von Hep-G2-Zellen ApoB-100-assoziierte Partikel vorhanden sind, dann können diese an die insolubilisierten Kan-anti-LDL-Ak binden, und es entsteht ein Immunkomplex, der an die Plastikoberfläche gebunden ist. Nicht gebundene Proteine werden durch Waschen entfernt. Der sich an der Plastikoberfläche befindliche Immunkomplex wird mit monoklonalen Antikörpern nachgewiesen, die nach Standardbedingungen gegen humanes LDL induziert und gereinigt worden waren. Diese Antikörper wurden mit dem Enzym Peroxidase konjugiert. Peroxidase setzt das farblose Substrat TMB in Gegenwart von H₂O₂ in ein gefärbtes Produkt um. Nach Ansäuerung des Reaktionsgemisches mit H₂SO₄ wird die spezifische Lichtabsorption bei 450 nm bestimmt, die ein Maß für die Menge von ApoB-100-assoziierten Partikeln ist, die von den HepG2-Zellen in den Kulturüberstand sezerniert worden waren.

Überraschenderweise hemmen die erfindungsgemäßen Verbindungen die Freisetzung der ApoB-100-assoziierten Partikel. Der IC₅₀-Wert gibt an, bei welcher Substanzkonzentration die Lichtabsorption im Vergleich zur Kontrolle (Lösemittelkontrolle ohne Substanz) um 50% inhibiert ist.

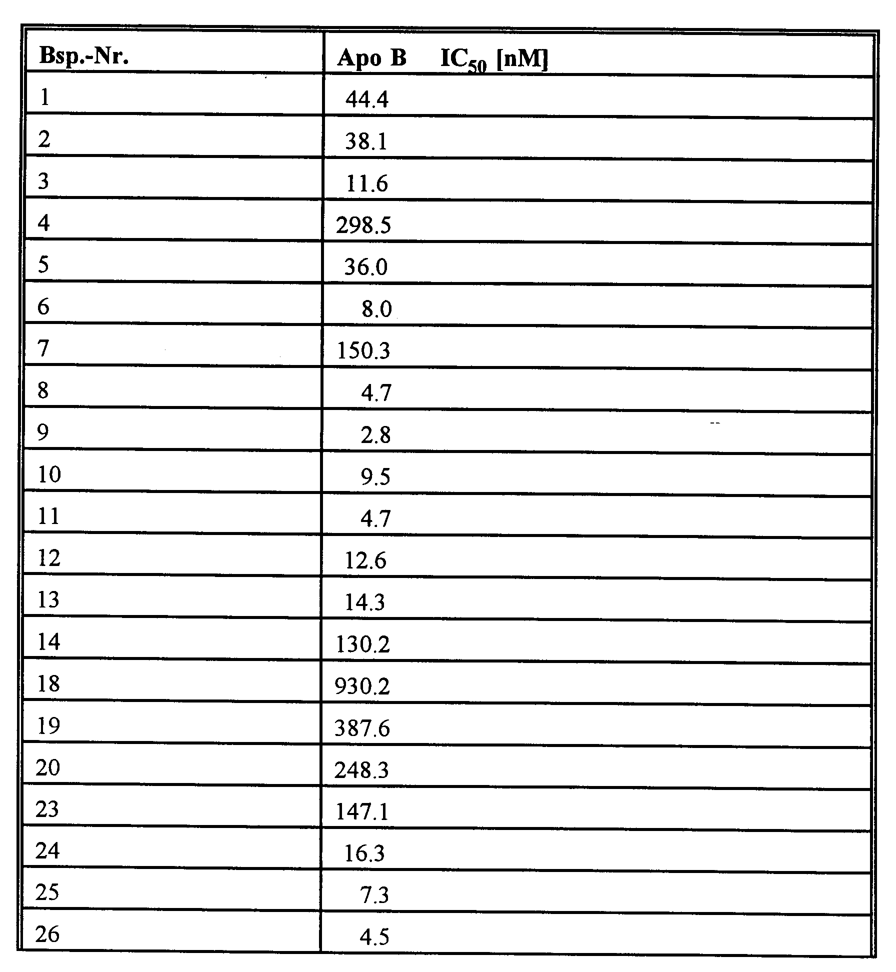

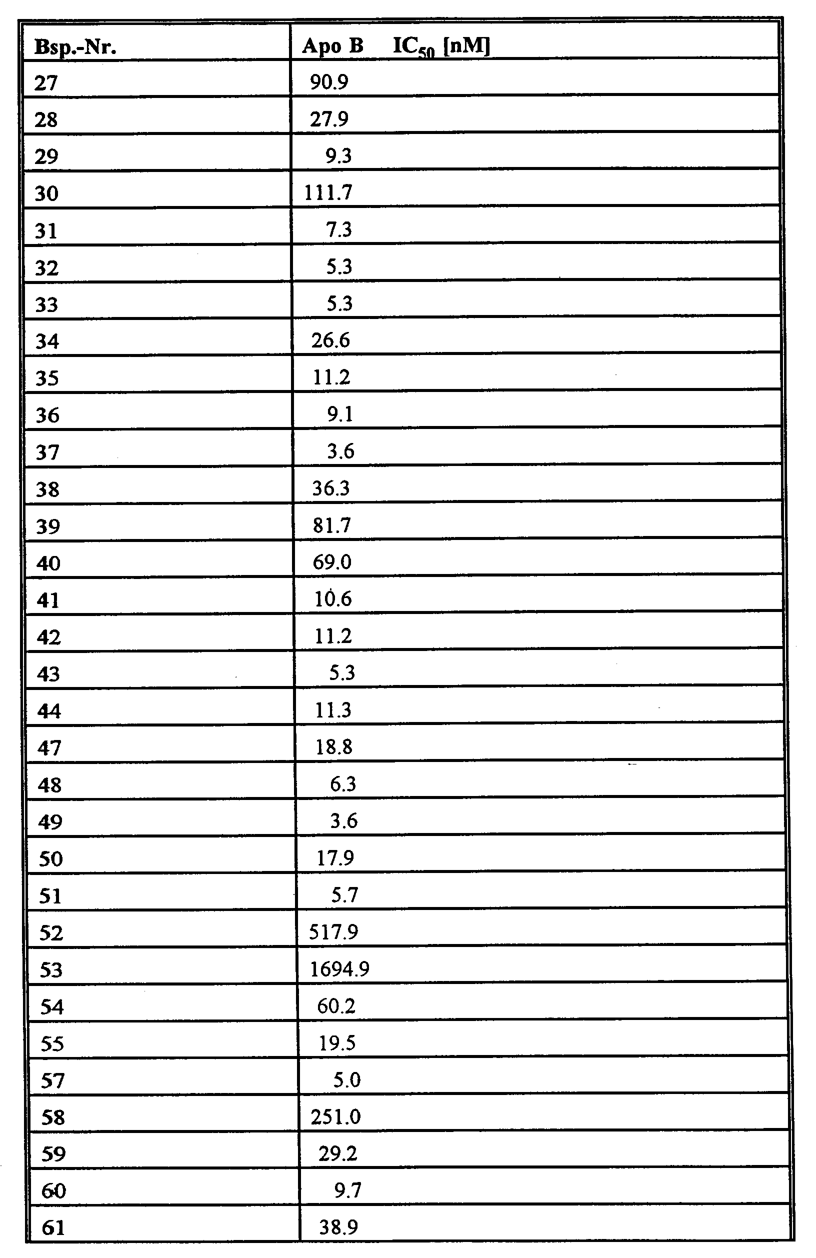

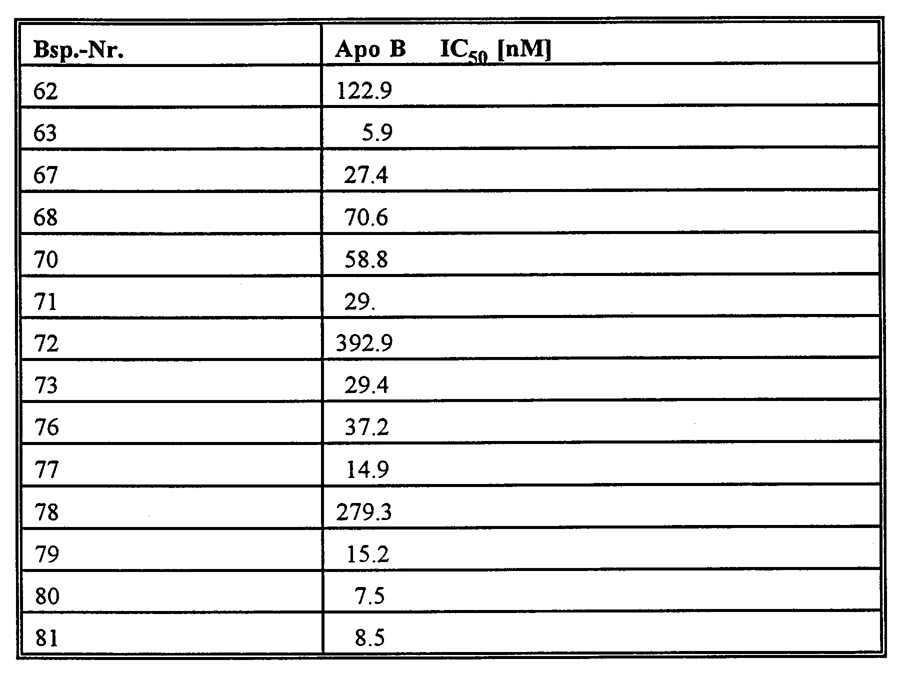

### 2. Bestimmung der VLDL-Sekretion in vivo am Hamster

Der Effekt der Testsubstanzen auf die VLDL-Sekretion in vivo wird am Hamster untersucht. Hierzu werden Goldhamster nach Prämedikation mit Atropin (83 mg/kg s.c.) mit Ketavet (83 mg/kg s.c.) und Nembutal (50 mg/kg i.p.) narkotisiert. Wenn die Tiere reflexfrei geworden sind, wird die V. jugularis freipräpariert und kanüliert. Anschließend werden 0,25 ml/kg einer 20%igen Lösung von Triton WR-1339 in physiologischer Kochsalzlösung appliziert. Dieses Detergens hemmt die Lipoproteinlipase und führt so zu einem Anstieg des Triglyceridspiegels aufgrund eines ausbleibenden Katabolismus von sezernierten VLDL-Partikeln. Dieser Triglyceridanstieg kann als Maß für die VLDL-Sekretionsrate herangezogen werden. Den Tieren wird vor sowie ein und zwei Stunden nach Applikation des Detergens durch Punktion des retroorbitalen Venenplexus Blut entnommen. Das Blut wird zwei Stunden bei Raumtemperatur, anschließend über Nacht bei 4°C inkubiert, um die Gerinnung vollständig abzuschließen. Danach wird 5 Minuten bei 10.000 g zentrifugiert. Im so erhaltenen Serum wird die Triglyceridkonzentration mit Hilfe eines modifizierten kommerziell erhältlichen Enzymtests bestimmt (Merckotest® Triglyceride Nr. 14354). 100 µl Serum werden mit 100 µl Testreagenz in 96-wird die optische Dichte bei einer Wellenlänge von 492 nm in einem automatischen Platten-Lesegerät bestimmt (SLT-Spectra). Serumproben mit einer zu hohen Triglyceridkonzentration werden mit physiologischer Kochsalzlösung verdünnt. Die in den Proben enthaltene Triglyceridkonzentration wird mit Hilfe einer parallel gemessenen Standardkurve bestimmt. Testsubstanzen werden in diesem Modell entweder unmittelbar vor Applikation des Detergens intravenös verabreicht oder vor Einleitung der Narkose oral oder subkutan.

### 3. Hemmung der intestinalen Triglyceridabsoprtion in vivo (Ratten)

Die Substanzen, die auf ihre triglyceridabsorptionshemmende Wirkung in vivo untersucht werden sollen, werden männlichen Wistar-Ratten mit einem Körpergewicht zwischen 170 und 230 g oral verabreicht. Zu diesem Zweck werden die Tiere 18 Stunden vor der Substanzapplikation in Gruppen zu 6 Tieren eingeteilt und anschließend wird ihnen das Futter entzogen. Trinkwasser steht den Tieren ad libitum zur Verfügung. Die Tiere der Kontrollgruppen erhalten eine wäßrige Traganth-Suspension bzw. eine Traganth-Suspension die Olivenöl enthält. Die Traganth-Olivenöl-Suspension wird mit dem Ultra-Turrax hergestellt. Die zu untersuchenden Substanzen werden in einer entsprechenden Traganth-Olivenöl-Suspension ebenfalls mit dem Ultra-Turrax, direkt vor der Substanzapplikation suspendiert.

Jeder Ratte wird vor der Schlundsondenapplikation zur Bestimmung des basalen Serumtriglyceridgehaltes Blut durch Punktion des retroorbitalen Venenplexus' entnommen. Anschließend werden die Traganth-Suspension, die Traganth-Olivenöl-Suspensionen ohne Substanz (Kontrolltiere), bzw. die Substanzen, suspendiert in einer entsprechenden Traganth-Olivenöl-Suspension, den nüchternen Tieren mit einer Schlundsonde verabreicht. Die weiteren Blutentnahmen zur Bestimmung des postprandialen Serumtriglyceridanstiegs erfolgen in der Regel 1, 2 und 3 Stunden nach der Schlundsondenapplikation.

Die Blutproben werden zentrifugiert und nach Gewinnung des Serums die Triglyceride photometrisch mit einem EPOS-Analyzer 5060 (Eppendorf Gerätebau, Netheler & Hinz GmbH, Hamburg) bestimmt. Die Bestimmung der Trigylceride erfolgt vollenzymatisch mit einem handelsüblichen UV-Test.

Der postprandiale Serumtriglyceridanstieg wird durch Subtraktion des Triglyceridvorwertes jeden Tieres von seinen korrespondierenden postprandialen Triglyceridkonzentrationen (1, 2 und 3 Stunden nach Applikation) ermittelt.

Die Differenzen (in mmol/l) zu jedem Zeitpunkt (1, 2 und 3 Stunden) werden in den Gruppen gemittelt, und die Mittelwerte des Serumtriglyceridanstiegs (ΔTG) der substanzbehandelten Tiere mit den Tieren verglichen, die nur die Traganth-Öl-Suspension erhielten.

Ebenso wird der Serumtriglyceridverlauf der Kontrolltiere, die nur Traganth erhielten, berechnet. Der Substanzeffekt zu jedem Zeitpunkt (1, 2 oder 3 Stunden) wird wie folgt ermittelt und in Δ% von der ölbelasteten Kontrolle angegeben.$\text{Δ% Triglyceridanstieg =} \frac{{\text{ΔTG}}_{\text{Substanz}} {\text{- ΔTG}}_{\text{Traganthkontrolle}}}{{\text{ΔTG}}_{\text{Ölbelastung}} {\text{- ΔTG}}_{\text{Traganthkontrolle}}} \text{x 100}$

Effekt von 10 mg Prüfsubstanz / kg KG p.o. auf den Triglyceridanstieg (Δ%) 2 h nach einer Triglyceridbelastung im Serum nüchterner Ratten. Der Serumtriglyceridanstieg fettbelasteter Kontrolltiere bezogen auf den Serumtriglyceridspiegel von Traganth-Kontrolltieren entspricht 100%. n = 6 Tiere pro Gruppe.

Die statistische Auswertung erfolgt mit Student's t-Test nach vorheriger Überprüfung der Varianzen auf Homogenität.

Substanzen, die zu einem Zeitpunkt den postprandialen Serumtriglyceridanstieg, verglichen mit dem der unbehandelten Kontrollgruppe, statistisch signifikant (p <0,05) um mindestens 30 % vermindern, werden als pharmakologisch wirksam angesehen.

| **Bsp.-Nr.** | **Hemmung der Intestinalen TG Absorption in vivo (Ratten)** |
|---|---|
| 3 | 2 mg/kg |
| 5 | 2-3 mg/kg |
| 6 | < 3 mg/kg |
| 8 | > 6 mg/kg |
| 9 | 2-4 mg/kg |
| 10 | 3 mg/kg |
| 21 | > 6 mg/kg |
| 22 | 7 mg/kg |
| 26 | 10 mg7kg |
| 29 | >/= 3 mg/kg |
| 30 | >>10 mg/kg |
| 32 | 3-6 mg/kg |
| 33 | 2 mg/kg |
| 34 | > 6 mg/kg |
| 38 | > 2 mg/kg |
| 39 | 2 mg/kg |
| 40 | >> 6 mg/kg |
| 41 | >> 2 mg/kg |
| 44 | > 2 mg/kg |
| 45 | 3 mg/kg |
| 46 | >> 2 mg/kg |
| 48 | 3 mg/kg |
| 51 | >> 10 mg/kg |
| 52 | 3 mg/kg |
| 54 | 6 mg/kg |
| 57 | 2 mg/kg |
| 60 | 6 mg/kg |
| 64 | >> 10 mg/kg |
| 68 | > 10 mg/kg |
| 70 | >> 2 mg/kg |
| 74 | >> 2 mg/kg |
| 77 | 8 mg/kg |

### 4. Hemmung der VLDL-Sekretion in vivo (Ratte)

Die Wirkung der Testsubstanzen auf die VLDL-Sekretion wird ebenfalls an der Ratte untersucht. Dazu wird Ratten 500 mg/kg Körpergewicht (2,5 mg/kg) Triton WR-1339, gelöst in physiologischer Kochsalzlösung, intravenös in die Schwanzvene appliziert. Triton WR-1339 inhibiert die Lipoproteinlipase und führt somit durch Hemmung des VLDL-Katabolismus zu einem Anstieg des Triglycerid- und Cholesterinspiegels. Diese Anstiege können als Maß für die VLDL-Sekretionsrate herangezogen werden.

Den Tieren wird vor sowie eine und zwei Stunden nach Applikation des Detergens durch Punktion des retroorbitalen Venenplexus Blut entnommen. Das Blut wird zur Gerinnung 1 h bei Raumtemperatur inkubiert und das Serum durch Zentrifugation mit 10 000 g für 20 s gewonnen. Anschließend werden die Triglyceride mittels eines handelsüblichen gekoppelten Enzymtests (Sigma Diagnostics®, Nr. 339) bei einer Wellenlänge von 540 nm photometrisch bestimmt. Die Messung erfolgt mit Hilfe eines ebenfalls gekoppelten Enzymtests (Boehringer Mannheim®, Nr. 1442350) bei einer Wellenlänge von 546 nm. Proben mit Triglycerid- bzw. Cholesterinkonzentrationen, die den Meßbereich der Methoden überschreiten, werden mit physiologischer Kochsalzlösung verdünnt. Die Ermittlung der jeweiligen Serumkonzentrationen erfolgt anhand parallel gemessener Standardreihen. Testsubstanzen werden unmittelbar nach der Tritoninjektion oral, intravenös oder subcutan appliziert.

Die Erfindung betrifft außerdem die Kombination von bicyclischen Heterocyclen der allgemeinen Formel (I) mit einem Glucosidase- und/oder Amylasehemmer zur Behandlung von familiärer Hyperlipidämien, der Fettsucht (Adipositas) und des Diabetes mellitus. Glucosidase- und/oder Amylasehemmer im Rahmen der Erfindung sind beispielsweise Acarbose, Adiposine, Voglibose (AO-128), Miglitol, Emiglitate, MDL-25637, Camiglibase (MDL-73945), Temdamistate, AI-3688, Trestatin, Pradimicin-Q und Salbostatin. Bevorzugt ist die Kombination von Acarbose, Miglitol, Emiglitate oder Vaglibose mit einer der oben aufgeführten erfindungsgemäßen Verbindungen der allgemeinen Formel (I).

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht-toxischer, pharmazeutisch geeigneter Trägerstoffe oder Lösemittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösemitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösemittel als Hilfslösemittel verwendet werden können.

Die Applikation erfolgt in üblicher Weise, vorzugsweise oral oder parenteral, insbesondere perlingual oder intravenös.

Für den Fall der parenteralen Anwendung können Lösungen des Wirkstoffs unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

### Verwendete Abkürzungen:

- CI: = Chemische Ionisation
- cHept: = cyclo Heptyl
- cHex: = cyclo Hexyl
- cPent: = cyclo Pentyl
- d: = doublet
- DCCI: = N'-(3-Dimethylaminopropyl)-N'-ethylcarbodiimid
- dd: = doublet doublets
- dia: = Diastereomer
- DMF: = N,N-Dimethylformamid
- DMSO: = Dimethylsulfoxid
- EI: = Elektronenstoß-Ionisation
- FAB: = Fast Atom Bombardment
- HOBT: = 1-Hydroxy-1H-benzotriazol
- Hz: = Hertz
- iBu: = iso Butyl
- iPr: = iso Propyl
- m: = multiplet
- Me: = Methyl
- nPr: = normales Propyl
- Ph: = Phenyl
- RT: = Raumtemperatur
- s: = singlet
- t: = triplet
- TFA: = Trifluoressigsäure
- THF: = Tetrahydrofuran
- TMS: = Tetramethylsilan

### Benutzte Solvenzgemische

| | | | | | |
|---|---|---|---|---|---|
| Petrolether : Aceton | | = | 1:1 | (A) | |
| Petrolether : Essigester | | = | 20:1 | (B) | |
| Petrolether : Essigester | | = | 10:1 | (C) | |
| Petrolether : Essigester | | = | 5:1 | (D) | |
| Petrolether : Essigester | | = | 3:1 | (E) | |
| Petrolether : Essigester | | = | 4:1 | (F) | |
| Petrolether : Essigester | | = | 2:1 | (G) | |
| Petrolether : Essigester | | = | 1:1 | (H) | |
| Petrolether : Essigester | | = | 1:2 | (I) | |
| Dichlormethan : Methanol | = | 50:1 | (J) | | |
| Dichlormethan : Methanol | = | 20:1 | (K) | | |
| Dichlormethan : Methanol | = | 10:1 | (L) | | |
| Dichlormethan : Essigester | = | 1:1 | (M) | | |
| Dichlormethan : Ethanol | | = | 50:1 | (N) | |
| Dichlormethan (100%) | | = | | (O) | |
| Essigester: Methanol | | | = | 10:1 | (P) |
| Toluol (100%) | | = | | (Q) | |
| Toluol : Essigester | | = | 1:1 | (R) | |
| Toluol : Essigester | | = | 8:1 | (S) | |
| Toluol : Essigester | | = | 9:1 | (T) | |
| Cyclohexanol : Essigester | = | 1:1 | (U) | | |
| Cyclohexanol : Essigester | = | 7:3 | (V) | | |

### Ergänzungen:

Wenn FAB nicht benutzt worden ist, haben folgende Kennzeichnungen in allen nachfolgenen Tabellen Gültigkeit:
* = EI
# = CI (NH₃)

### Beispiel I

### 2-(R&S)-Phenyl-2-(4-methyl)phenylessigsäuremethylester

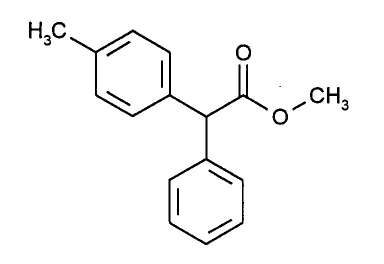

21.0 g (100 mmol) 2-Phenyl-1-(4-methyl)phenyl-1-oxoethane und 38.8 g (120 mmol) Iodbenzoldiacetat wurden in 300 ml Orthoameisensaeuretrimethylester gelöst. Zu dieser Lösung wurden 19.6 g konz. Schwefelsäure gegeben, und die Lösung wurde für 6 Std auf 60°C erwärmt. Die Lösung wurde auf Raumtemperatur abgekühlt, mit Wasser (100 mL) verdünnt, und mit Diethylether extrahiert. Die vereinigten organischen Phasen wurden über Natriumsulfat getrocknet und einrotiert. Der Rückstand wurde säulenchromatographisch gereinigt:
Ausbeute 13.1 g (55%);
R_{f} = 0.33 (Petrolether : Essigester, 20:1);
Masse (berechnet) für C₁₆H₁₆O₂ = 240.30; Massenspektrum (FAB, rel. Intensität) 241 (25%), 181 (100%);
¹H NMR (200 MHz, CDCl₃) δ 7.3-7.10 (m, 9 H), 4.99 (s, 1 H), 3.73 (s, 3 H), 2.31 (s, 3 H).

### Beispiel II

### 2-Cyclopentyl-2-(4-methylphenyl)-essigsäure-tert.butylester

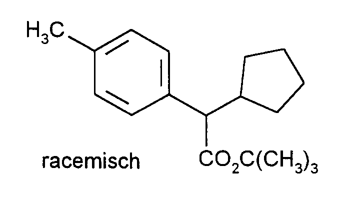

33.5 g (0.3 mol) Kalium-tert.butylat werden in 100 ml wasserfreiem DMF bei 0°C vorgelegt, und 51.6 g (0.25 mol) 4-Methylphenyl-essigsäure-tert.butylester in 250 ml wasserfreiem DMF zugetropft. Es wird 30 min. bei 0°C gerührt und 32.2 ml (0.3 mol) Cyclopentylbromid in 150 ml wasserfreiem DMF bei 5-15°C zugetropft und 20 h bei 25°C gerührt. Nach Einengen wird der Rückstand zwischen Wasser und Diethylether verteilt, die Etherphase über Natriumsulfat getrocknet und eingeengt. Das Produkt kristallisiert aus.
Ausbeute: 67 g (97.5% d.Th.);
Festpunkt: 51-53°C.

Die Verbindungen der Tabelle I werden analog der Vorschrift des Beispiels II hergestellt:

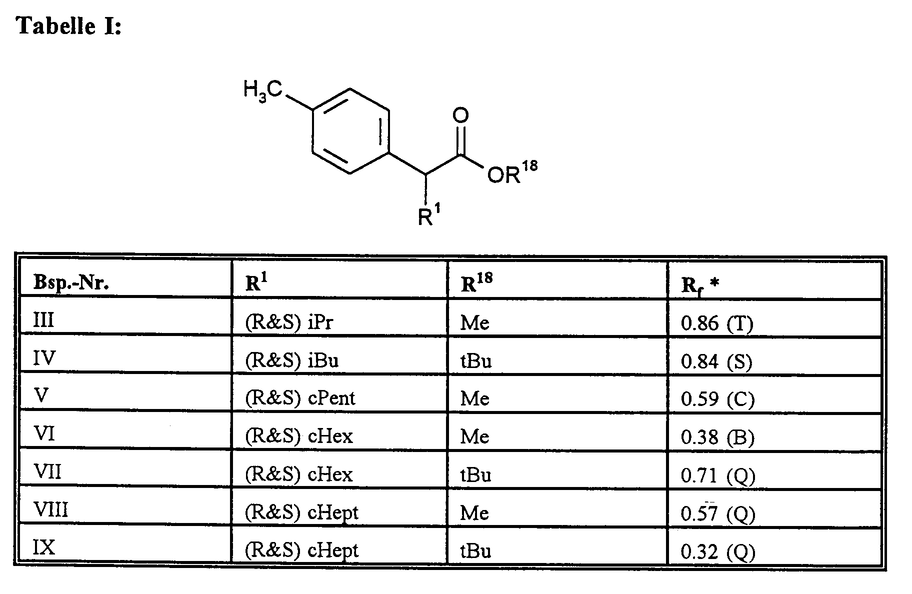

### Beispiel X

### 2-(4-Brommethyl-phenyl)-2-cyclopentyl-essigsäure-tert.butylester

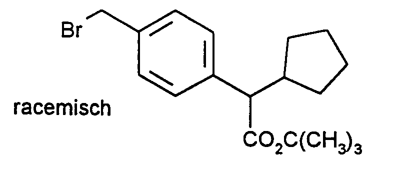

27.4 g (0.1 mol) der Verbindung aus Beispiel II werden in 200 ml Tetrachlormethan gelöst und zum Sieden erhitzt. Nach Zugabe von 0.82 g Azobisisobutyronitril werden 18.7 g (0.105 mol) N-Bromsuccinimid portionsweise zugegeben und anschließend 1 h refluxiert, auf 0°C abgekühlt und vom Succinimid abfiltriert. Nach Einengen des Filtrats fällt das Produkt aus. Es wird mit Petrolether (40/60) gewaschen und getrocknet.
Ausbeute: 20 g (57% d.Th.);
Fp.: 73-76°C.

Die Verbindungen der Tabelle II werden analog der Vorschrift des Beispiels X hergestellt:

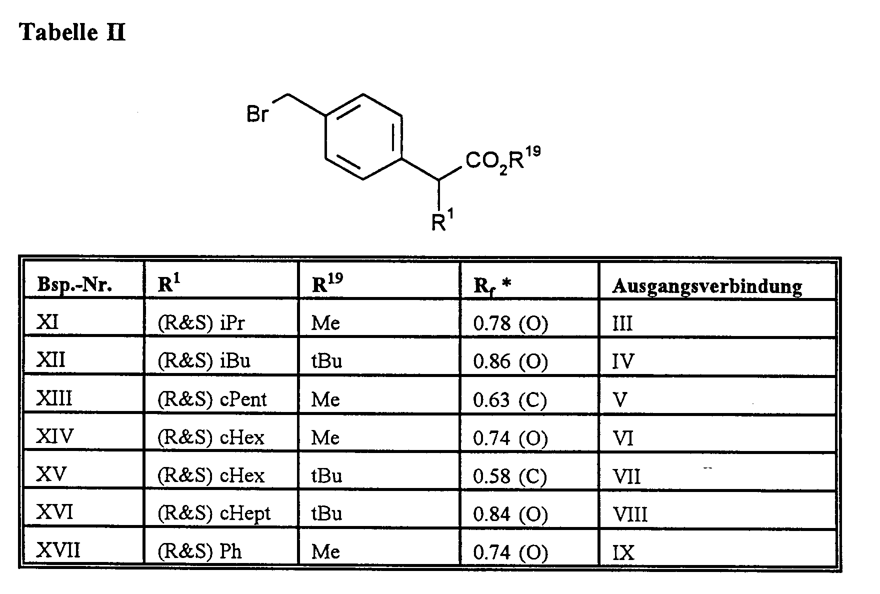

### Beispiel XVIII

### 4-Carboethoxy-2-phenylthiazol

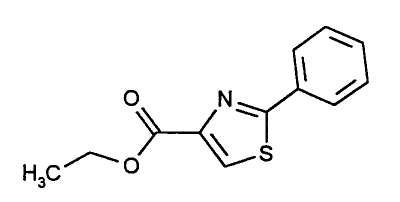

Man löst 13.19 g (96 mmol) Thiobenzamid in 260 ml THF. Dazu tropft man eine Lösung aus 25.0 g (115.37 mmol, 90%ig) Brombrenztraubensäureethylester in 250 ml THF bei Raumtemperatur zu. Die Temperatur steigt dabei auf 35°C an. Das Gemisch wird über Nacht zum Rückfluß gekocht, einrotiert und der Rückstand wird mit Essigester und ges. NaHCO₃-Lösung extrahiert. Die organische Phase wird über Natriumsulfat getrocknet und einrotiert. Der Rückstand wird säulenchromatographisch gereinigt. (Kieselgel Merck 60 (0.040-0.063)):
Ausbeute: 20.1 g (89.6 %);
R_{f} = 0.66 (Petrolether:Essigester 3:1);
Masse (berechnet) für C₁₂H₁₁NO₂ = 233.29; Massenspektrum (CI, rel. Intensität) 234 (100%);
¹H NMR (200 MHz, CDCl₃) δ 8.15 (s, 1 H), 8.03-7.99 (m, 2 H), 7.48-7.42 (m, 3 H), 4.46 (q, J = 7.11 Hz, 2 H), 1.43 (t, J = 7.16 Hz, 3 H).

### Beispiel XIX

### 4-Carboxy-2-phenylthiazol

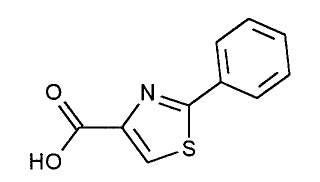

20.1 g (86.2 mmol) der Verbindung aus Beispiel XVII werden in 400 ml Ethanol gelöst. Dazu gibt man eine Lösung aus 17.2 g (430.8 mmol) Natriumhydroxidplätzchen in 80 ml Wasser hinzu. Es wird 6 Stunden zum Rückfluß gekocht. Es wird mit Wasser versetzt und unter Eiskühlung mit halbkonzentrierter Salzsäure auf pH 6 gestellt. Es wird einrotiert, und der Rückstand wird mit Essigester und Wasser extrahiert. Die organische Phase wird über Natriumsulfat getrocknet und einrotiert.
Ausbeute: 13.6 g (76.9%);
Fp = 170-172°C ;
R_{f} = 0.11 (CH₂Cl₂:Methanol, 20:1) ;
Masse (berechnet) für C₁₀H₇NO₂S = 205.24; Massenspektrum (EI, rel. Intensität) 205 (100%);
¹H NMR (250 MHz, DMSO-D₆) δ 13.2 (bs, 1 H), 8.52 (s, 1 H), 8.01-7.96 (m, 2 H), 7.60-7.45 (m, 3 H).

### Beispiel XX

### 2-[4-(2-phenyl)thiazolyl]benzimidazol

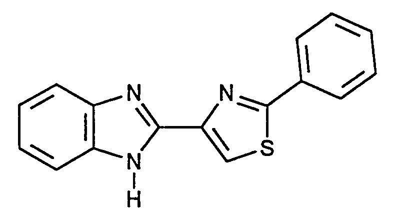

Man vermischt 13.3 g (64.8 mmol) der Verbindung aus Beispiel XIX und 7.0 g (64.8 mmol) Diaminobenzol miteinander. Unter ständigen Rühren mit einem Overheadrührer wird langsam 70 ml Polyphosphorsäure zugegeben. Es wird 1 Stunde auf 100°C und 6 Stunden auf 140°C erhitzt. Man gibt unter Eiskühlung Wasser und Natriumhydroxidplätzchen hinzu, und stellt den pH Wert auf 12 ein. Es wird mit Essigester versetzt und extrahiert. Die organische Phase wird über Natriumsulfat getrocknet und einrotiert. Der Rückstand wird mit CH₂Cl₂ verrieben, abgesaugt und getrocknet.
Ausbeute: 4.3 g (23.9 %);
Fp = 183-191°C ;
R_{f} = 0.51 (Petrolether : Essigester, 1:1) ;
Masse (berechnet) für C₁₆H₁₁N₃S = 277.35; Massenspektrum (EI, rel. Intensität) 277 (85%), 174 (100%);
¹H NMR (250 MHz, DMSO-D₆) δ 12.9 (bs, 1 H), 8.44 (s, 1 H), 8.16-8.09 (m, 2 H), 7.63-7.52 (m, 5 H), 7.27-7.22 (2 H).

In Analogie zur Vorschrift des Beispiels XX wurden die in der Tabelle III aufgeführten Verbindungen hergestellt:

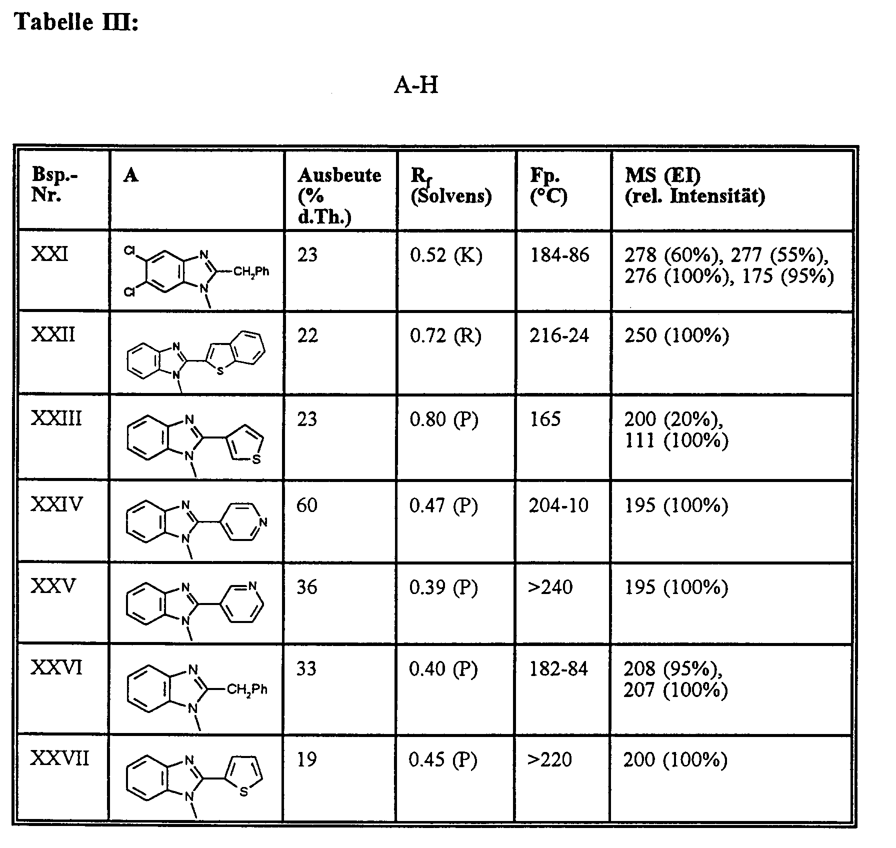

### Beispiel XXVIII

### 2-Cyclopentyl-2-[4-(2-methyl-4-oxo-4H-quinazolin-3-yl-methyl)phenyl]essigsäure-tert.butylester

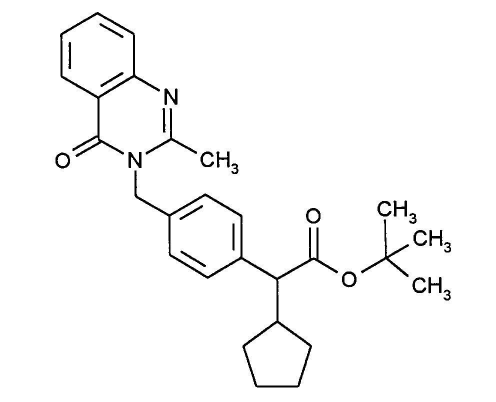

Es werden 374 mg (9.34 mmol) Natriumhydrid (60%ig in Paraffin) in 1.0 ml DMF vorgelegt. Man kühlt auf 0°C ab und tropft langsam eine Lösung aus 1.50 g (9.34 mmol) 2-Methyl-4(3H)-chinazolinon in 15 ml DMF dazu. Es wird eine halbe Stunde nachgerührt, wobei die Temperatur auf 20 °C steigt. Man kühlt wieder ab und löst 3.0 g (8.49 mmol) der Verbindung aus Beispiel II in 20 ml DMF und tropft diese zu. Die Mischung rührt über Nacht bei Raumtemperatur. Man gießt Wasser dazu und extrahiert (3x) mit Ether. Die vereinigten organischen Phasen werden über Natriumsulfat getrocknet und einrotiert. Der Rückstand wird säulenchromatographisch gereinigt (Kieselgel Merck 60 (0.040-0.063)):
Ausbeute: 2.88 g (78.3 %);
Fp = 128°C;
R_{f} = 0.54 (CH₂Cl₂:Methanol, 100:5);
Masse (berechnet) für C₂₇H₃₂N₂O₃ = 432.567; Massenspektrum (FAB, rel. Intensität) 433 (100%), 377 (100%), 376 (40%);
¹H NMR (250 MHz, CDCl₃) δ 8.31 (dd, J = 7.59 Hz, J = 1.25 Hz, 1 H), 7.77 (m, 1 H), 7.63 (d, J = 8.0 Hz, 1 H), 7.47 (m, 1 H), 7.28 (d, J = 8.22 Hz, 2 H), 7.14 (d, J = 8.26 Hz, 2 H), 5.37 (s, 2 H), 3.12 (d, J = 11.06 Hz, 1 H), 2.54 (s, 3 H), 2.47 (m, 1 H), 1.90 (m, 1 H), 1.65-1.10 (m, 6 H), 1.38 (s, 9 H), 0.92 (m, 1 H);
¹³C NMR (50 MHz, CDCl₃) δ 173.02 (s), 162.41 (s), 154.63 (s), 147.36 (s), 139.16 (s), 134.40 (d), 134.37 (s), 128.74 (d), 127.12 (d), 126.72 (d), 126.59 (d), 126.51 (d), 120.39 (s), 80.55 (s), 58.60 (d), 46.89 (t), 43.67 (d), 31.24 (t), 30.77 (t), 27.96 (q), 25.13 (t), 24.82 (t), 23.48 (q).

In Analogie zur Vorschrift des Beispiels XXVIII werden die in der Tabelle IV aufgeführten Verbindungen hergestellt:

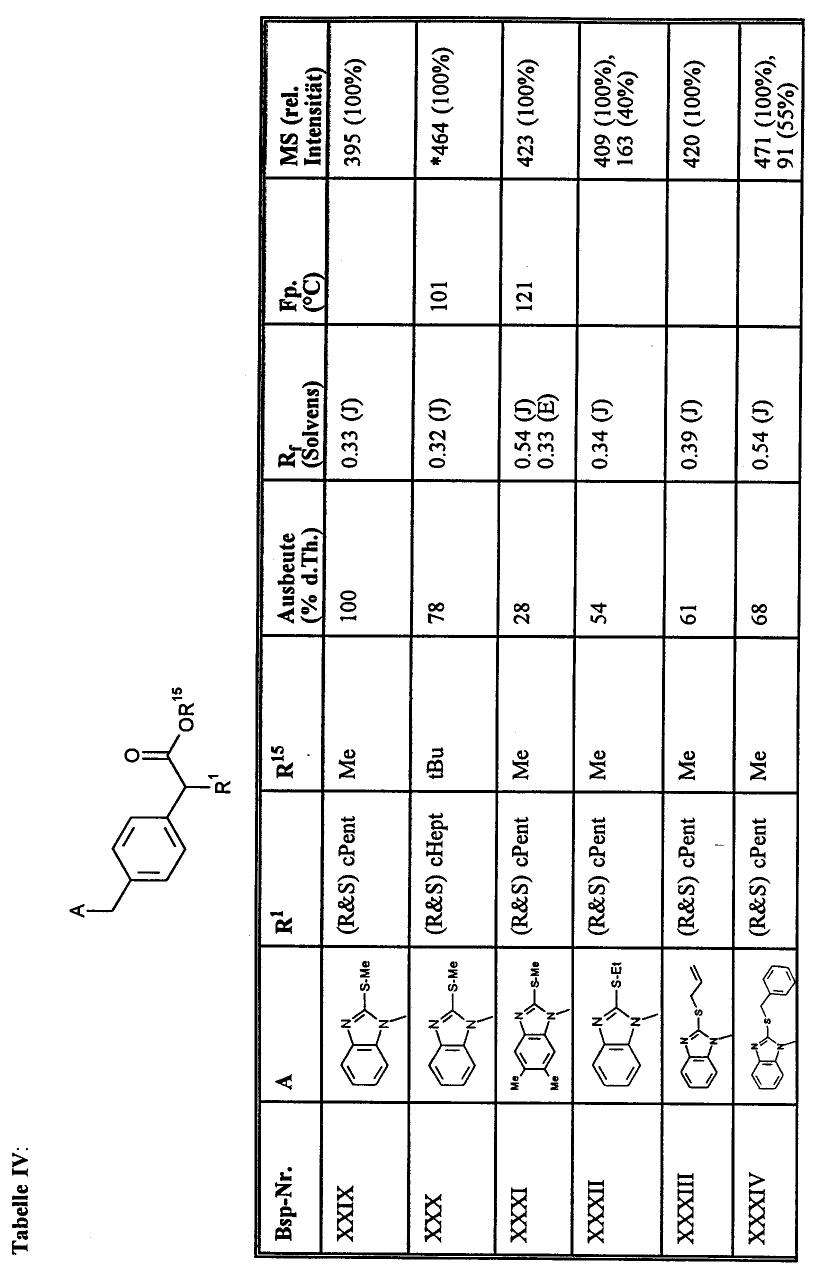

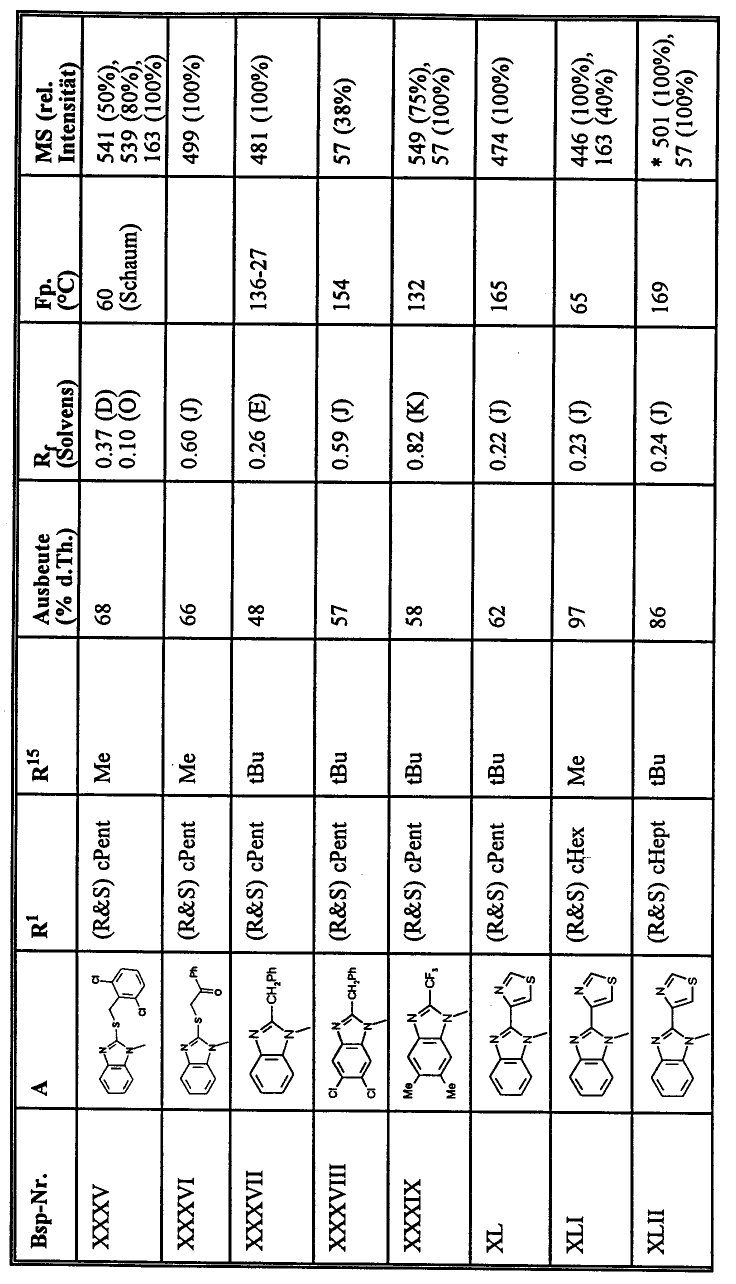

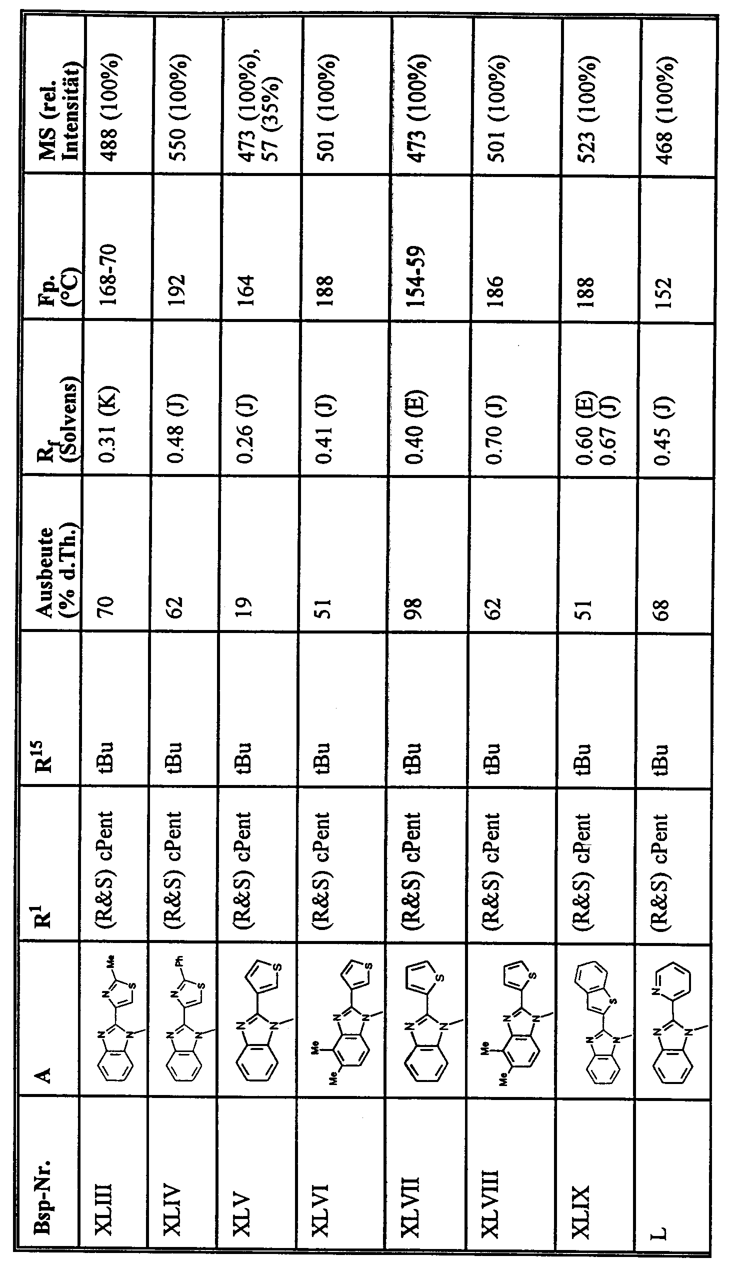

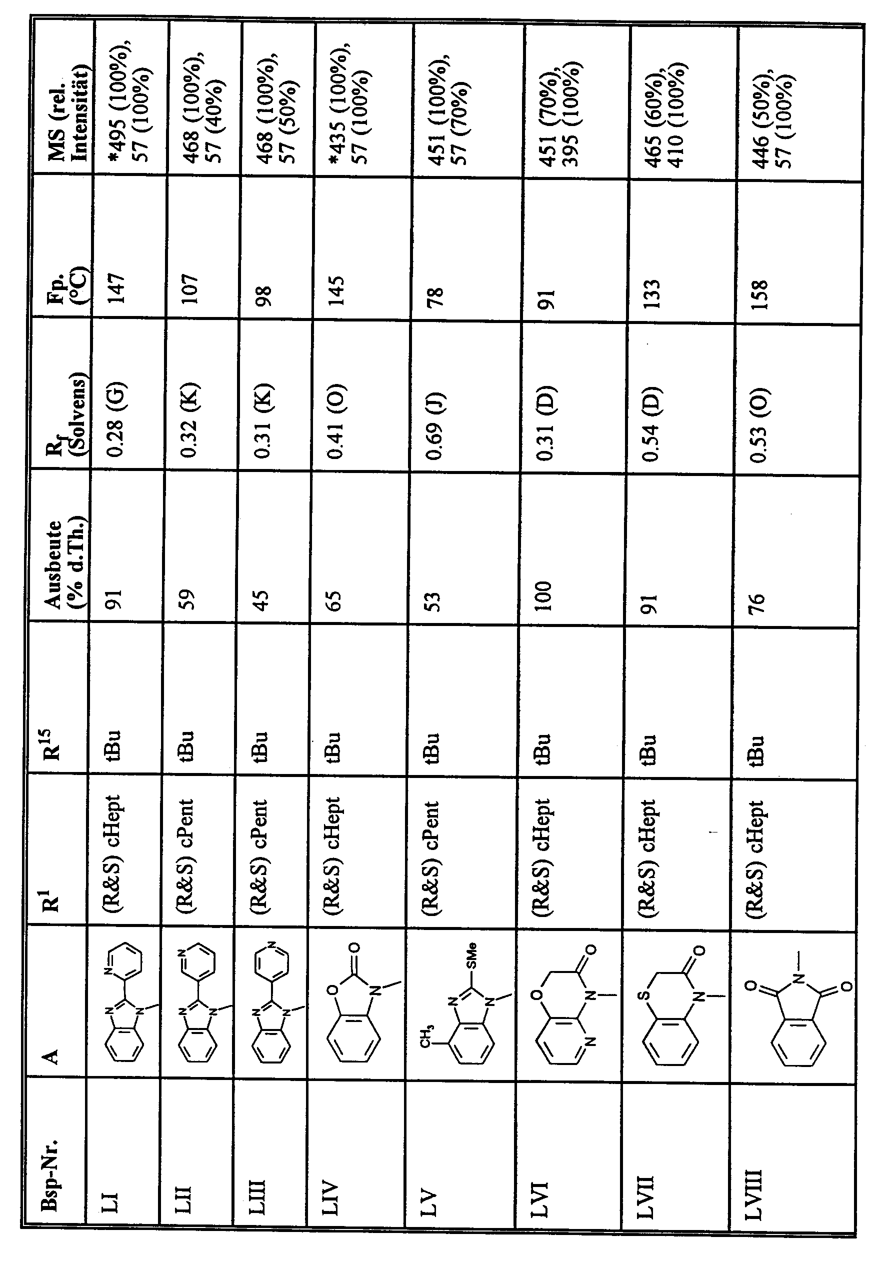

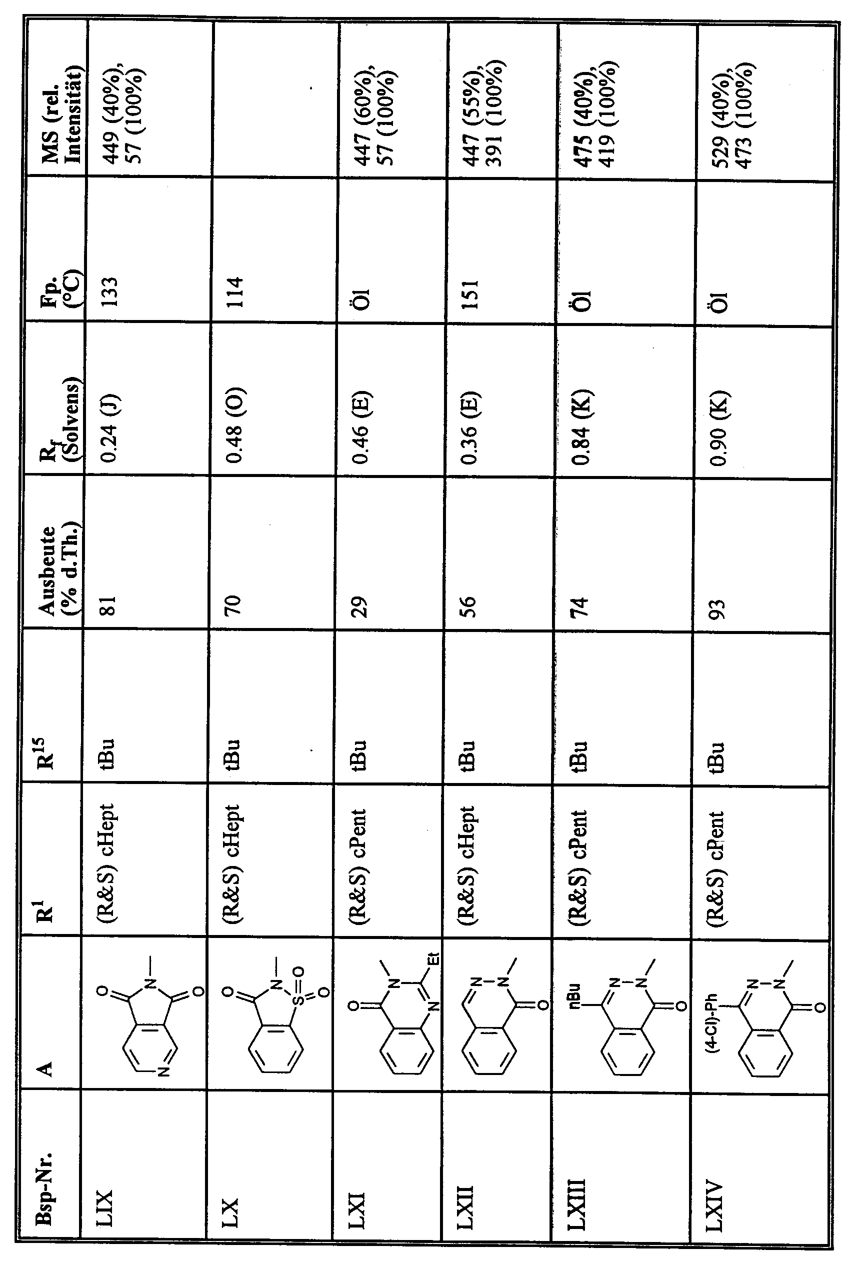

### Beispiel LXV

### Cyclopentyl-[4-(4-methyl-2-thioxo-2,3-dihydro-benzoimidazol-1-ylmethyl)phenyl]essigsäure

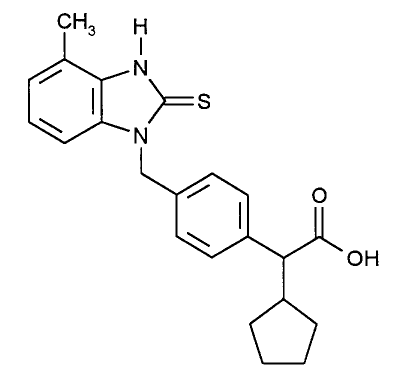

Man löst 1.93 g (4.29 mmol) der Verbindung aus Beispiel LV in 20 ml Dioxan und versetzt die Lösung mit 1 ml konzentrierter Salzsäure. Man läßt über Nacht zum Rückfluß kochen, kühlt ab und versetzt mit 25 ml kaltem Wasser. Der entstandene Niederschlag wird abgesaugt und getrocknet.
Ausbeute: 1.28 g (78.6 %);
Fp = >225°C ;
R_{f} = 0.35 (CH₂Cl₂:Methanol, 100:5) ;
Masse (berechnet) für C₂₂H₂₄N₂O₂S = 380.51

### Beispiel LXVI

### Cycloheptyl-[4-(2-thioxo-2,3-dihydro-benzoimidazol-1-ylmethyl)phenyl]essigsäure

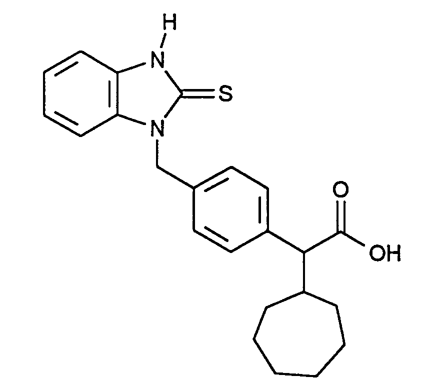

Die Reaktion wird mit 1.30 g der Verbindung aus Beispiel XXX analog der Vorschrift des Beispiel LXV durchgeführt.
Ausbeute: 162 mg (15%);
Fp = 245°C ;
R_{f} = 0.73 (Petrolether: Essigester, 2:1).

### Beispiel LXVII

### 2-Cyclopentyl-2-[4-(2-methyl-4-oxo-4H-quinazolin-3-yl-methyl)phenyl] essigsäure

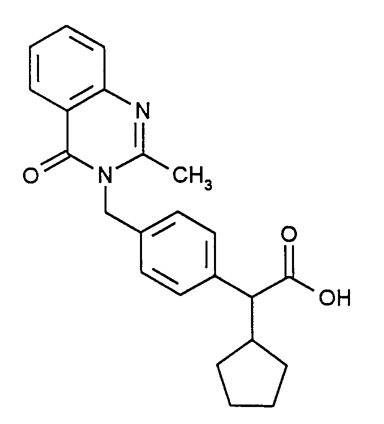

In 25 ml Dioxan wird 2.8 g (6.47 mmol) der Verbindung aus Beispiel XXXVII gelöst. Dazu gibt man 1.4 ml konzentrierte Salzsäure und kocht über Nacht zum Rückfluß. Man gibt Wasser und CH₂Cl₂ dazu und extrahiert. Die organische Phase wird über Natiumsulfat getrocknet und einrotiert. Der Rückstand wird säulenchromatographisch gereinigt (Kieselgel Merck 60 (0.040-0.063)):
Ausbeute: 1.68 g (68.9 %);
Fp = 181°C (Schaum);
R_{f} = 0.39 (CH₂Cl₂:Methanol 100:5);
Masse (berechnet) für C₂₃H₂₄N₂O₃ = 376.459: Massenspektrum (FAB, rel. Intensität) 378 (40%), 377 (100%);
¹H NMR (200 MHz, CDCl₃) δ 8.31 (dd, J = 7.74 Hz, J = 1.06 Hz, 1 H), 7.80-7.62 (m, 2 H), 7.45 (m, 1 H), 7.29 (d, J = 8.24 Hz, 2 H), 7.12 (d, J = 8.17 Hz, 2 H), 5.36 (s, 2 H), 3.26 (d, J = 11.12 Hz, 1 H), 2.51 (m, 1 H), 2.50 (s, 3 H), 1.95 (m, 1 H), 1.70-1.20 (m, 6 H), 0.97 (m, 1 H).

Analog den Vorschriften der Beispiele LXV - LXVII werden die in der Tabelle V aufgeführten Verbindungen hergestellt:

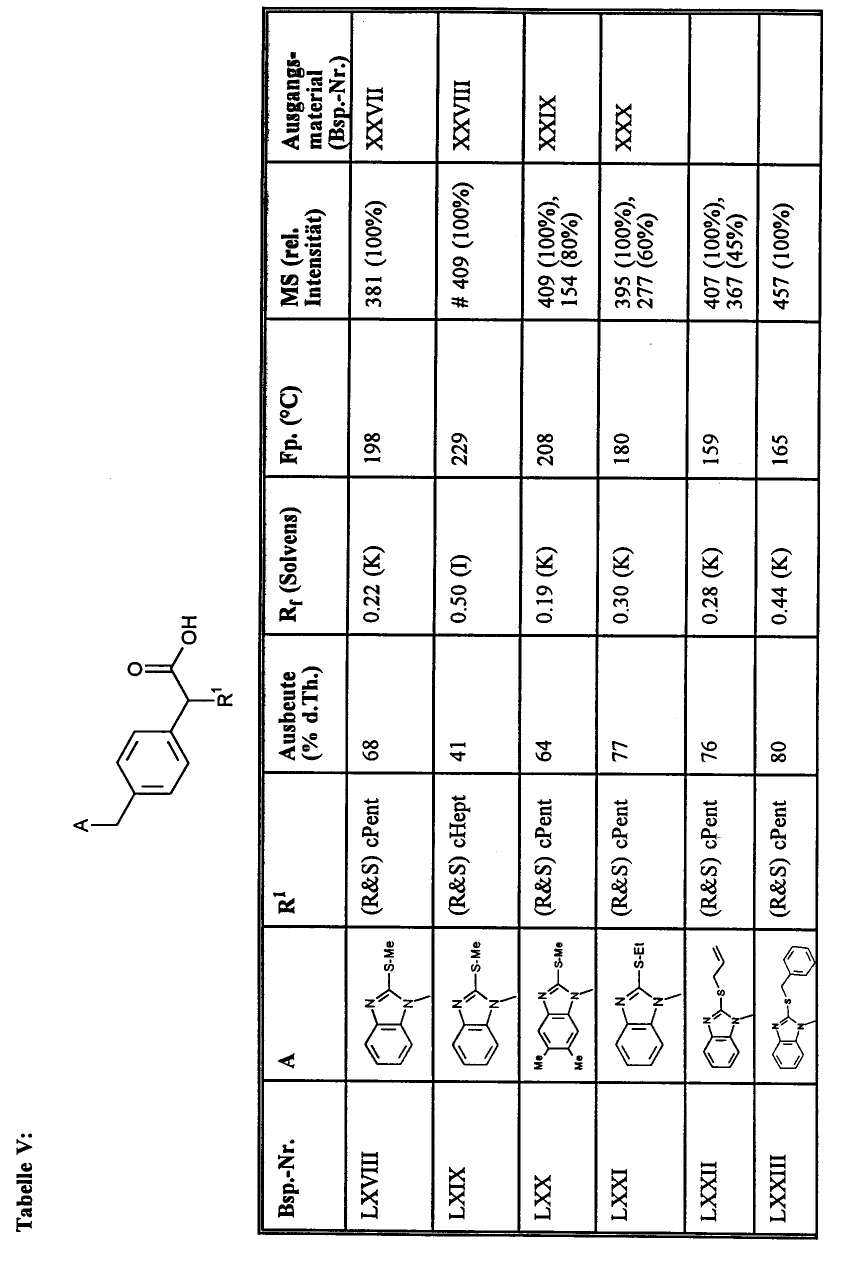

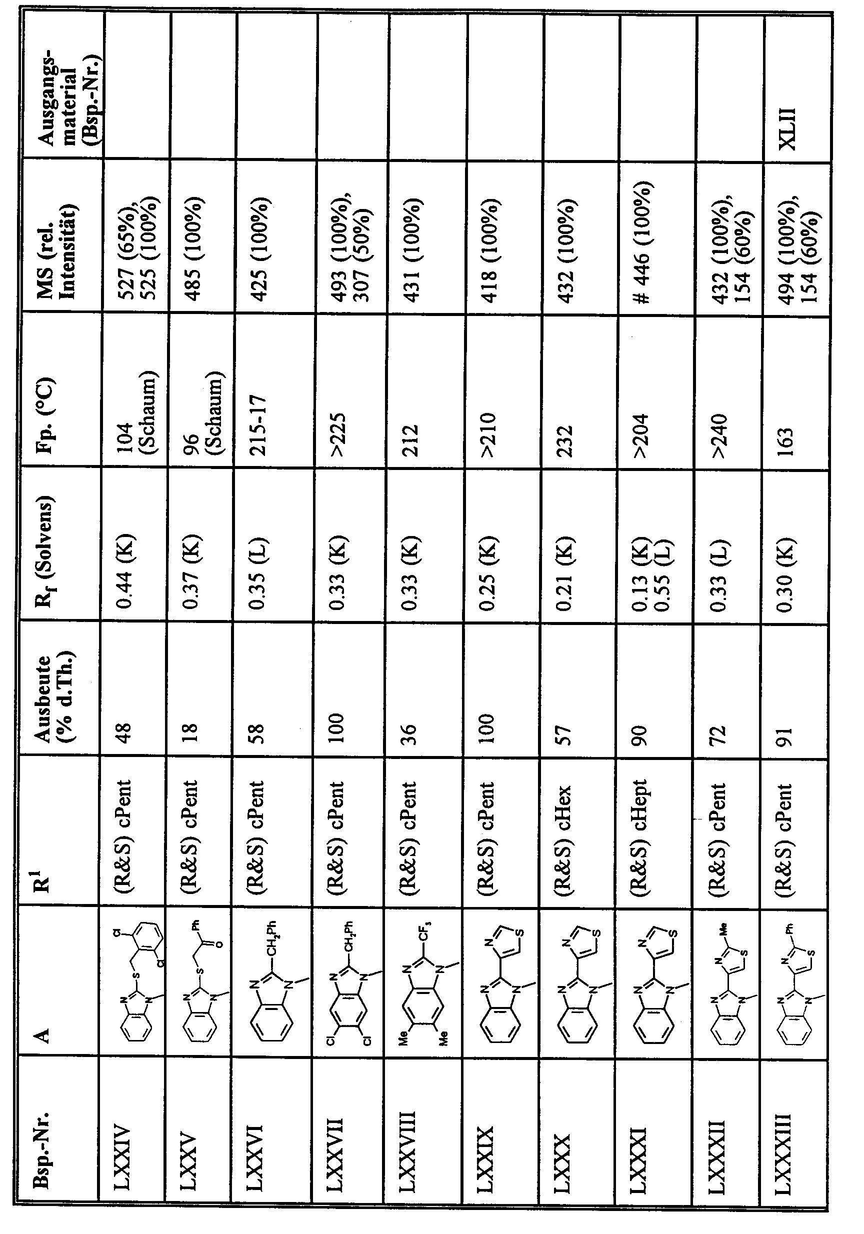

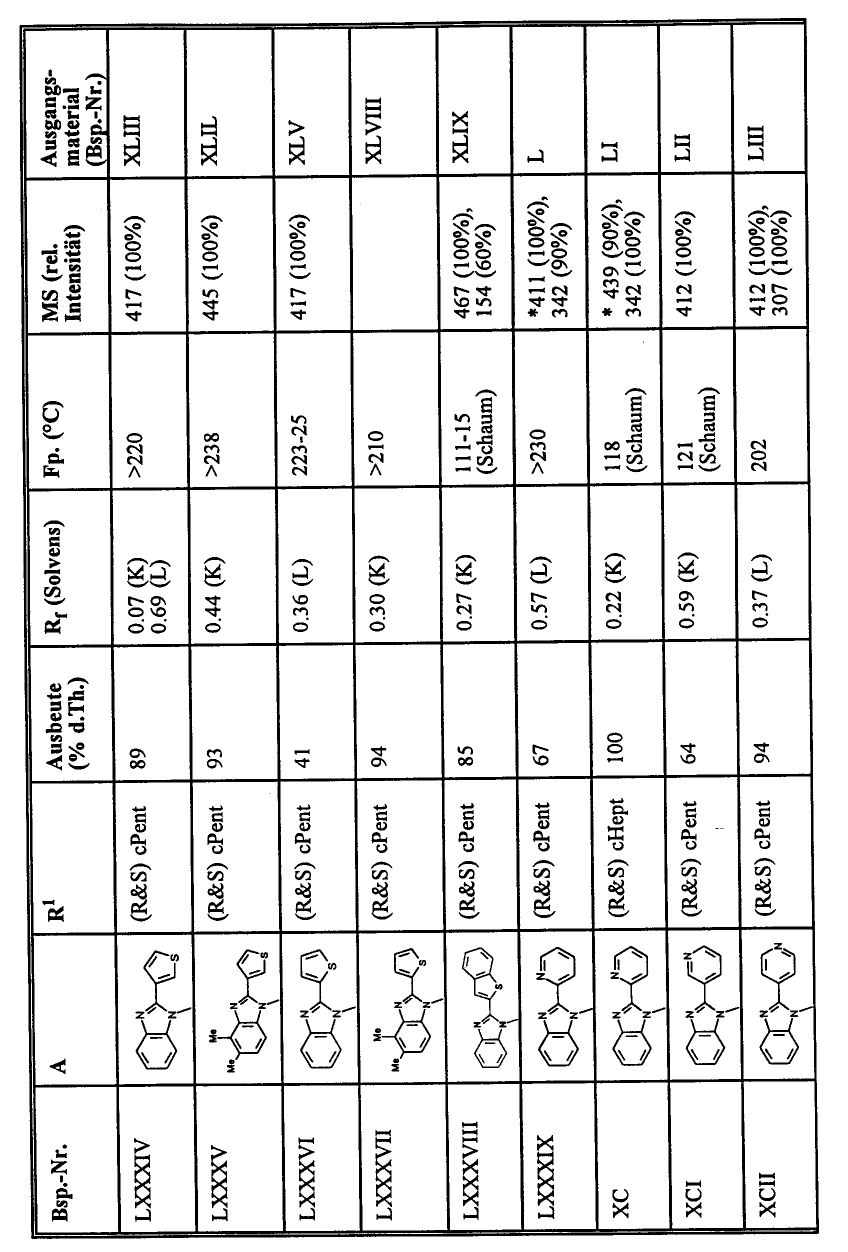

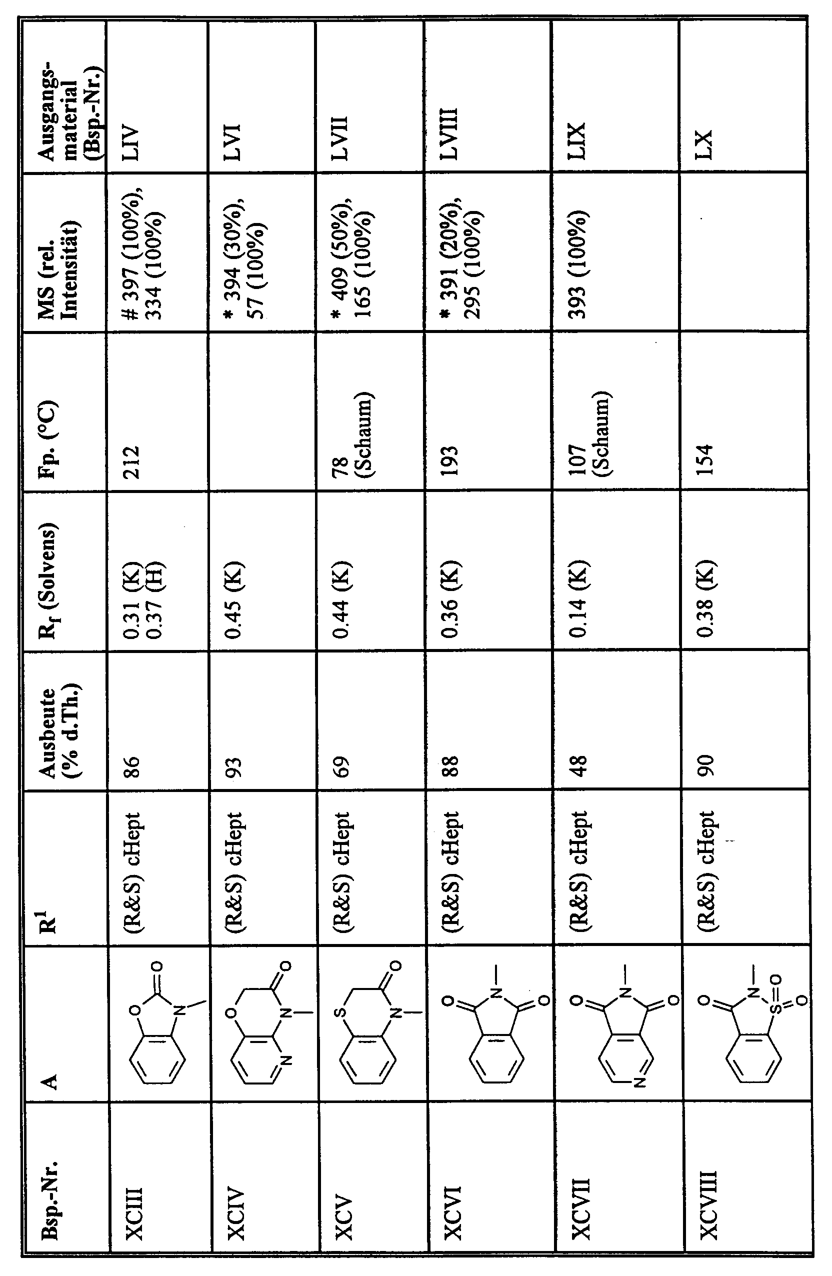

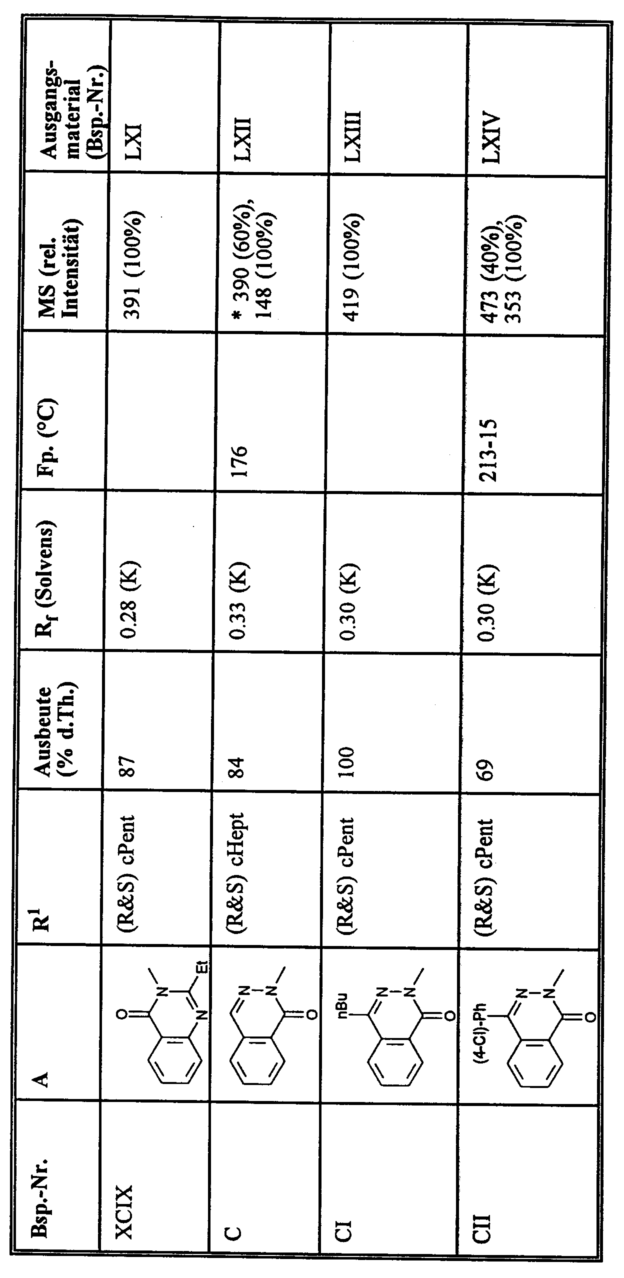

### Herstellungsbeispiele

### Beispiel 1

### 2-Cyclopentyl-N-(2-hydroxy-1-(R)-phenylethyl)-2-[4-(2-methyl-4-oxo-4H-quinazolin-3-yl-methyl)phenyl]essigsäureamid

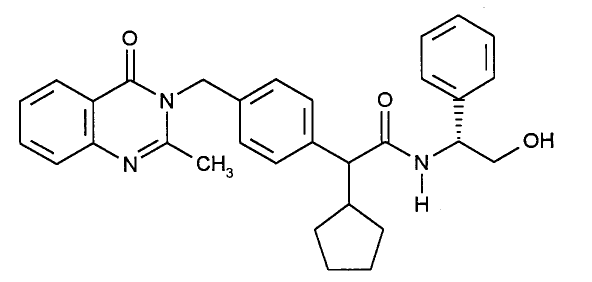

Man löst 1.645 g (4.37 mmol) der Verbindung aus Beispiel LXVII in 20 ml CH₂Cl₂. Dazu gibt man nacheinander 599 mg (4.37 mmol) R-Phenylglycinol, 649 mg (4.81 mmol) 1-Hydroxy-1-benzotriazol, 963 mg (5.02 mmol) N'(3-Dimethylaminopropyl)-N-ethylcarbodiimid x HCl und 884 mg (8.74 mmol) Triethylamin. Es wird über Nacht bei Raumtemperatur nachgerührt. Man verdünnt die Lösung mit 20 ml CH₂Cl₂ und extrahiert diese mit ges.NH₄Cl-Lösung und ges.NaHCO₃-Lösung. Die organische Phase wird über Natiumsulfat getrocknet und einrotiert. Der Rückstand wird säulenchromatographisch gereinigt (Kieselgel Merck 60 (0.040-0.063):
Ausbeute: 1.11 g (51.0 %);
Fp = 97°C (Schaum);
R_{f} = 0.50 (CH₂Cl₂:Methanol, 100:5);
Masse (berechnet) für C₃₁H₃₃N₃O₃ = 495.627; Massenspektrum (FAB, rel. Intensität) 496 (100%), 307 (40%).

In Analogie zur Vorschrift des Beispiels 1 werden die in der Tabelle 1 aufgeführten Verbindungen hergestellt:

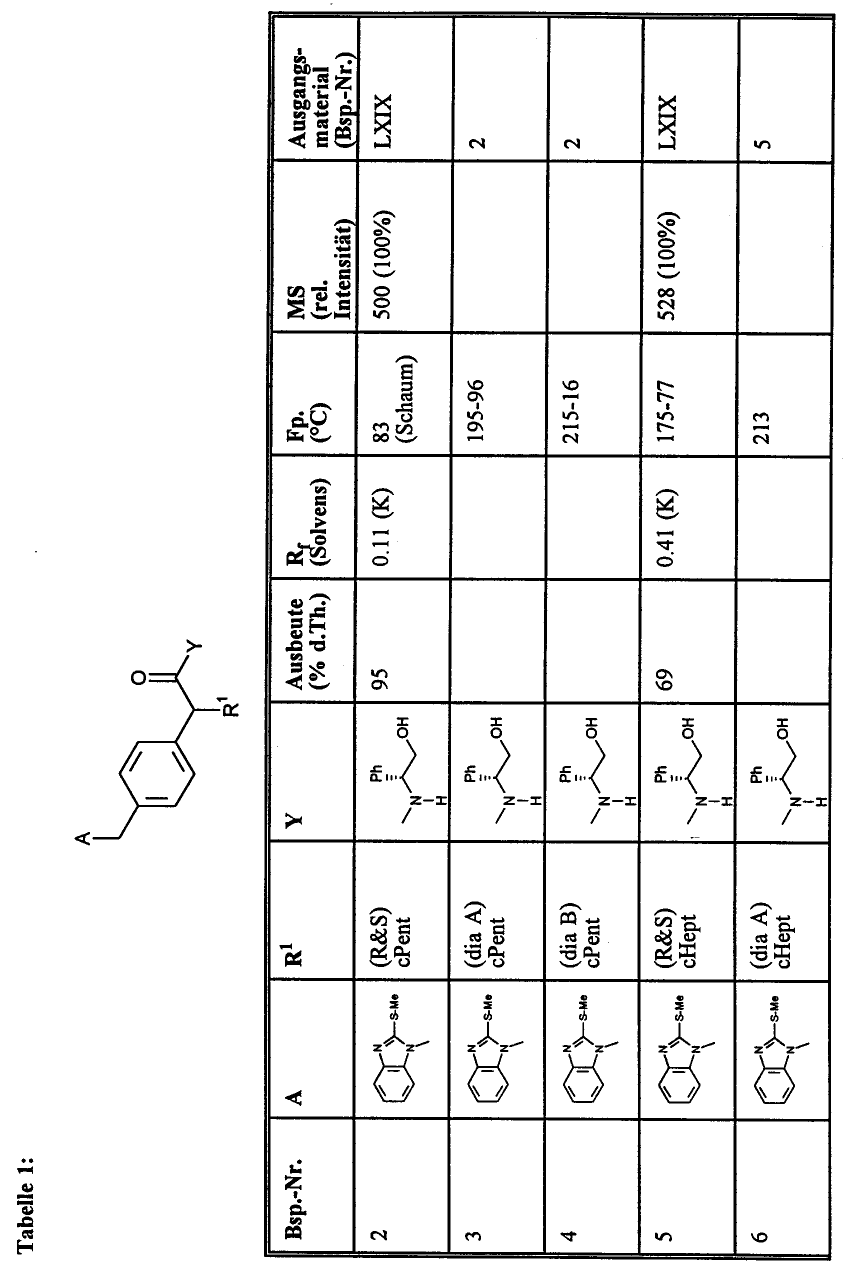

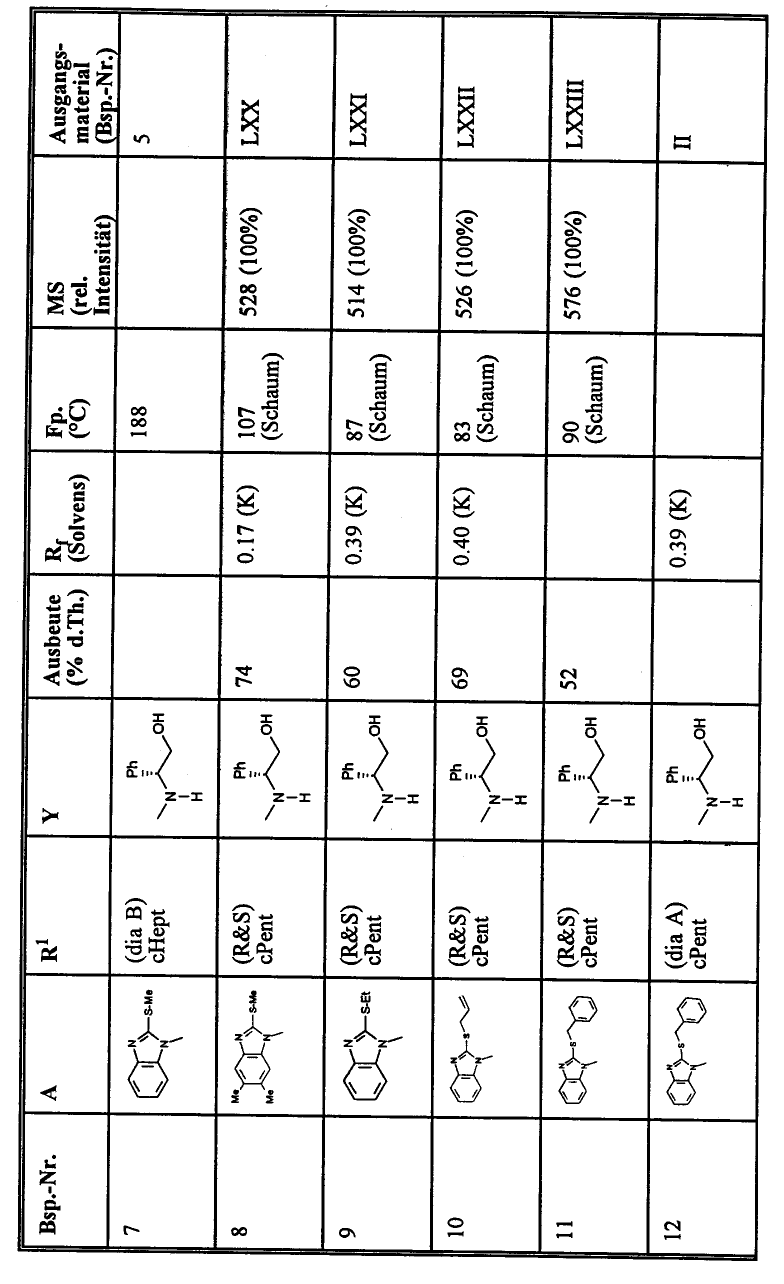

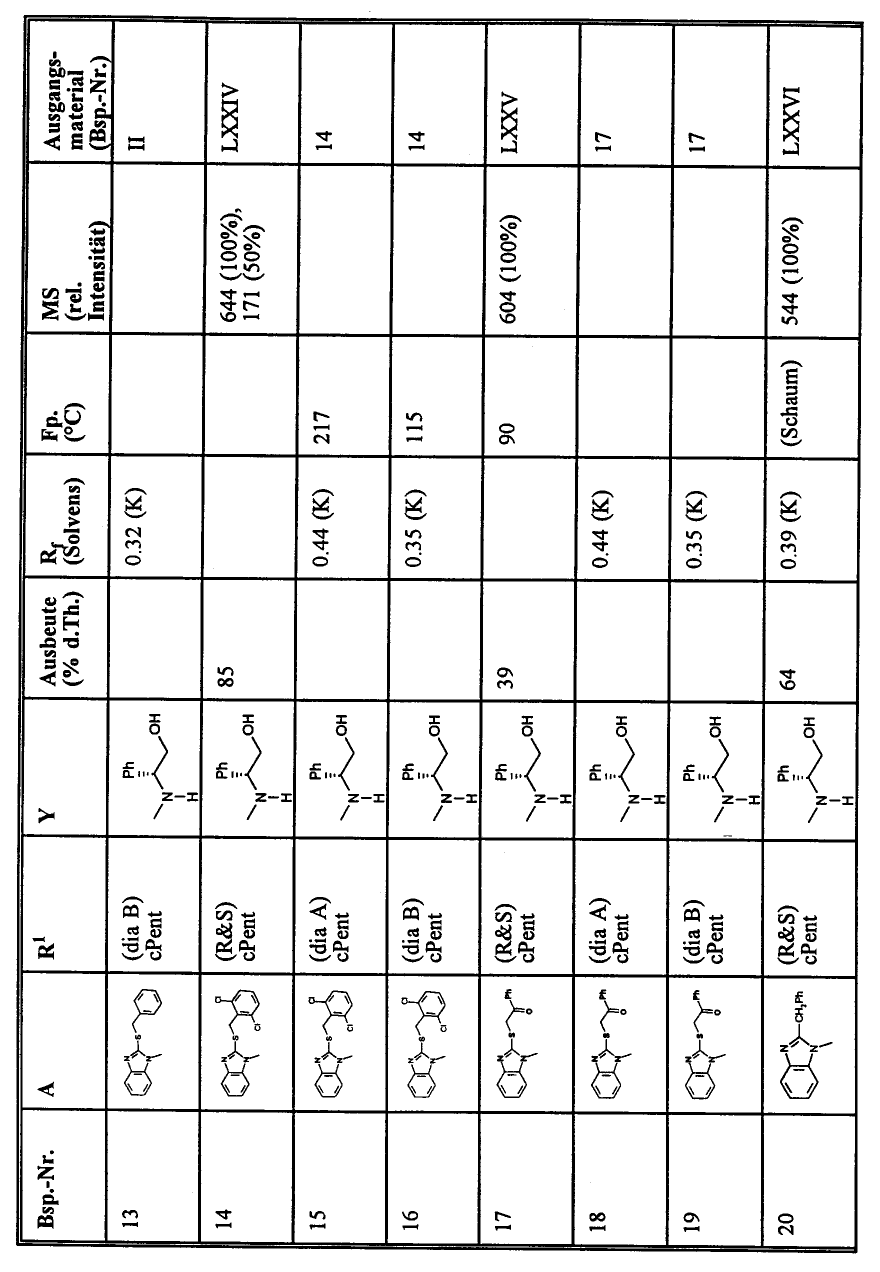

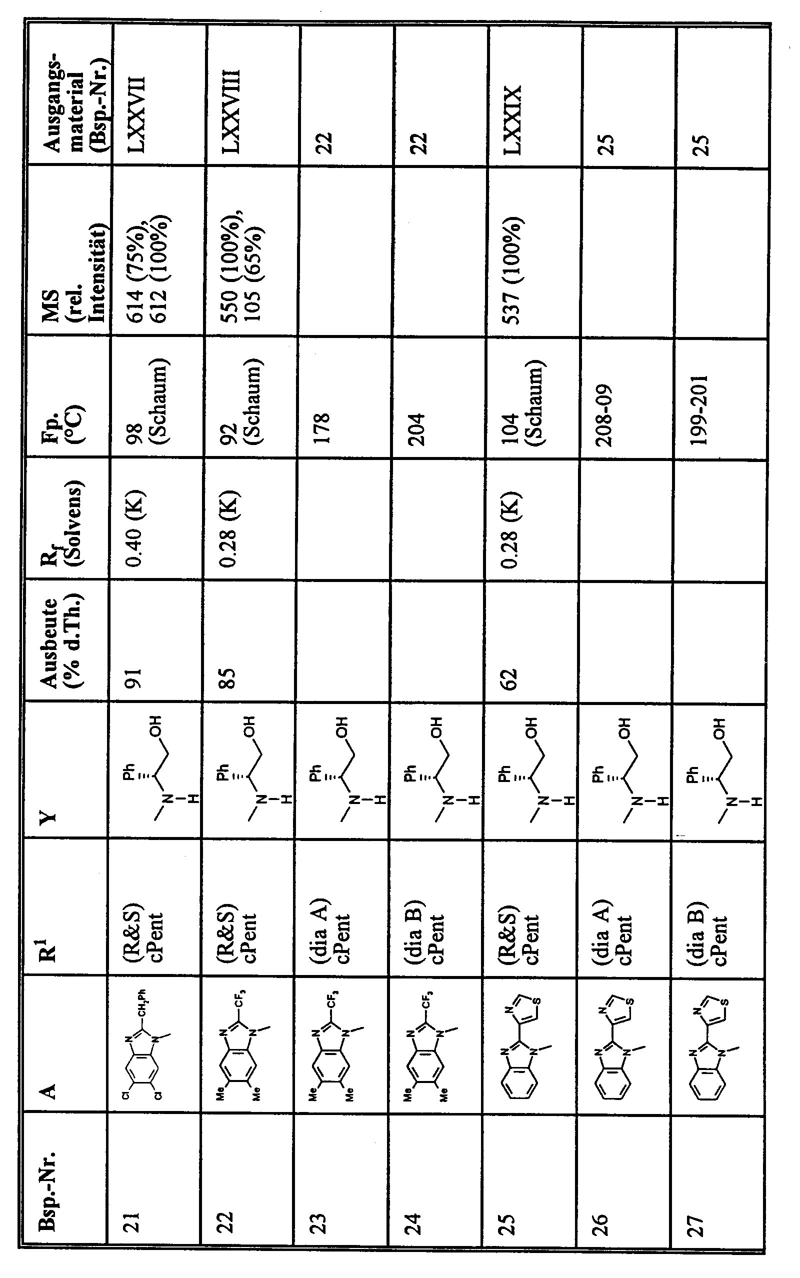

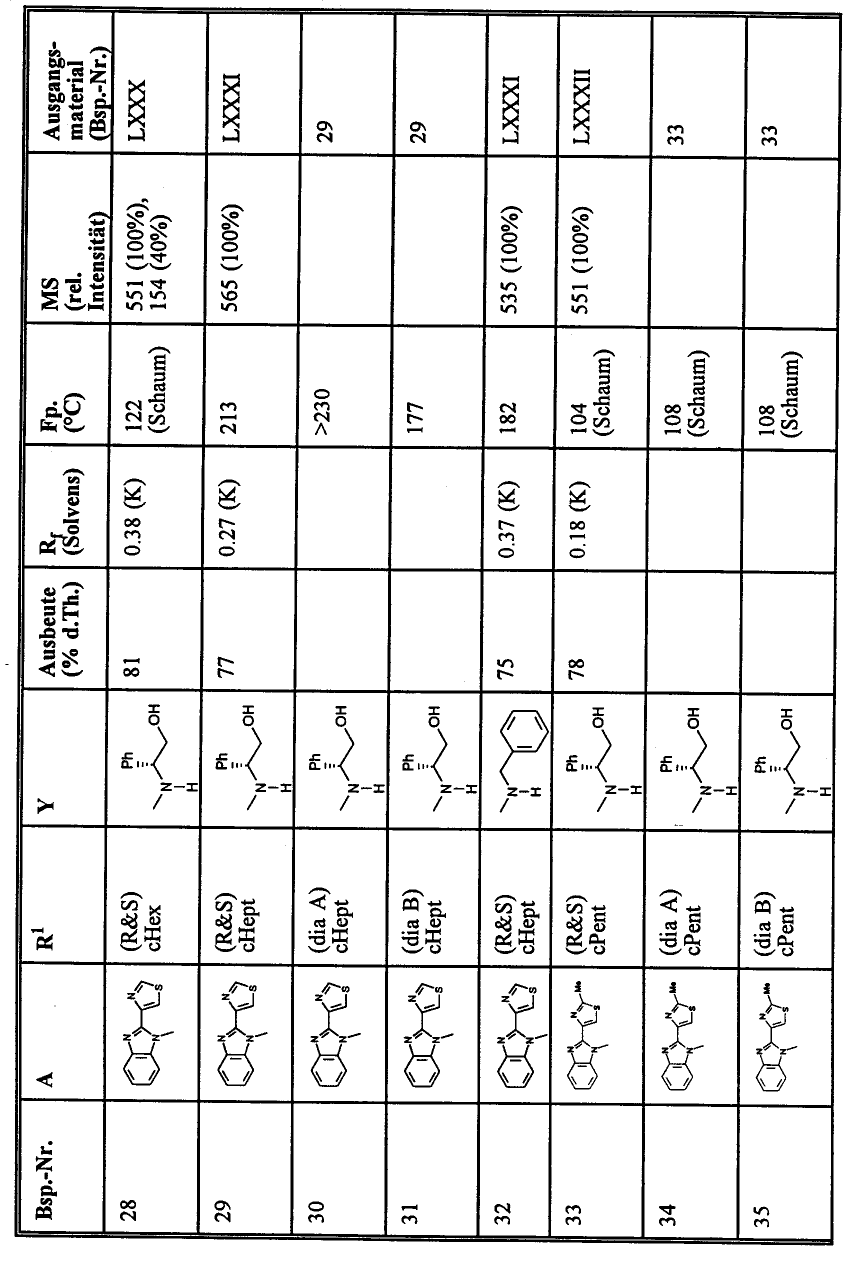

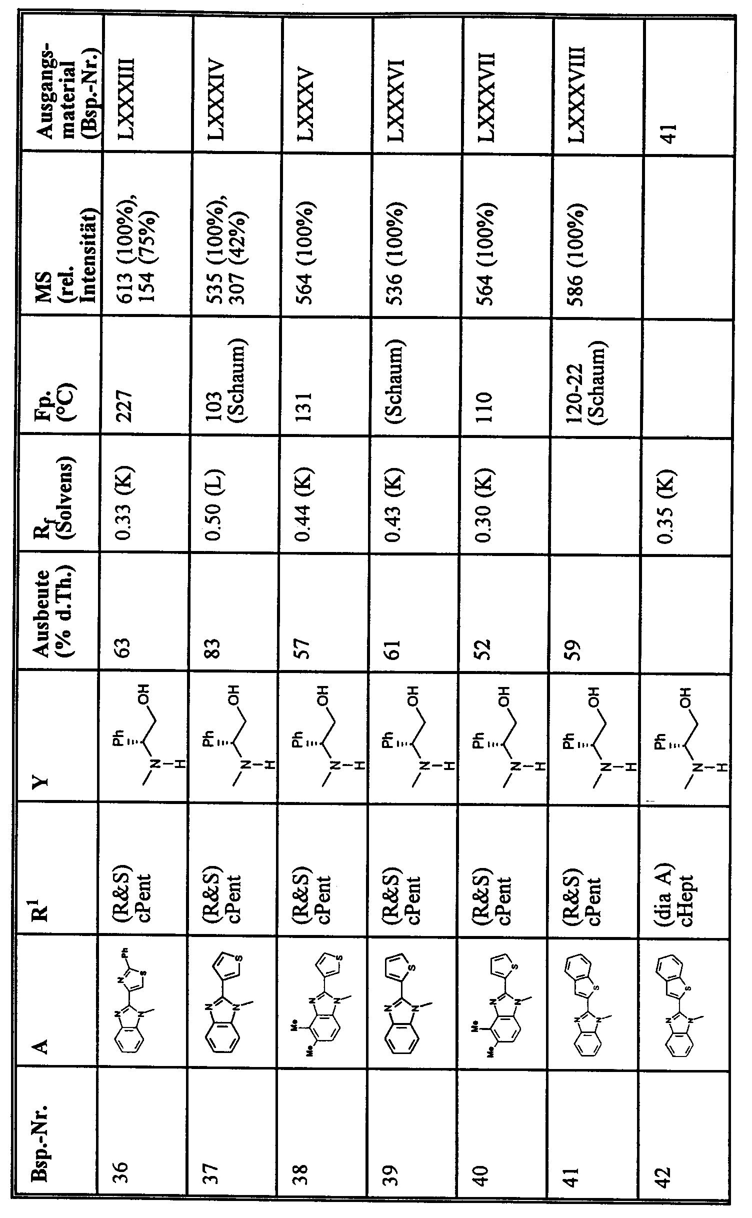

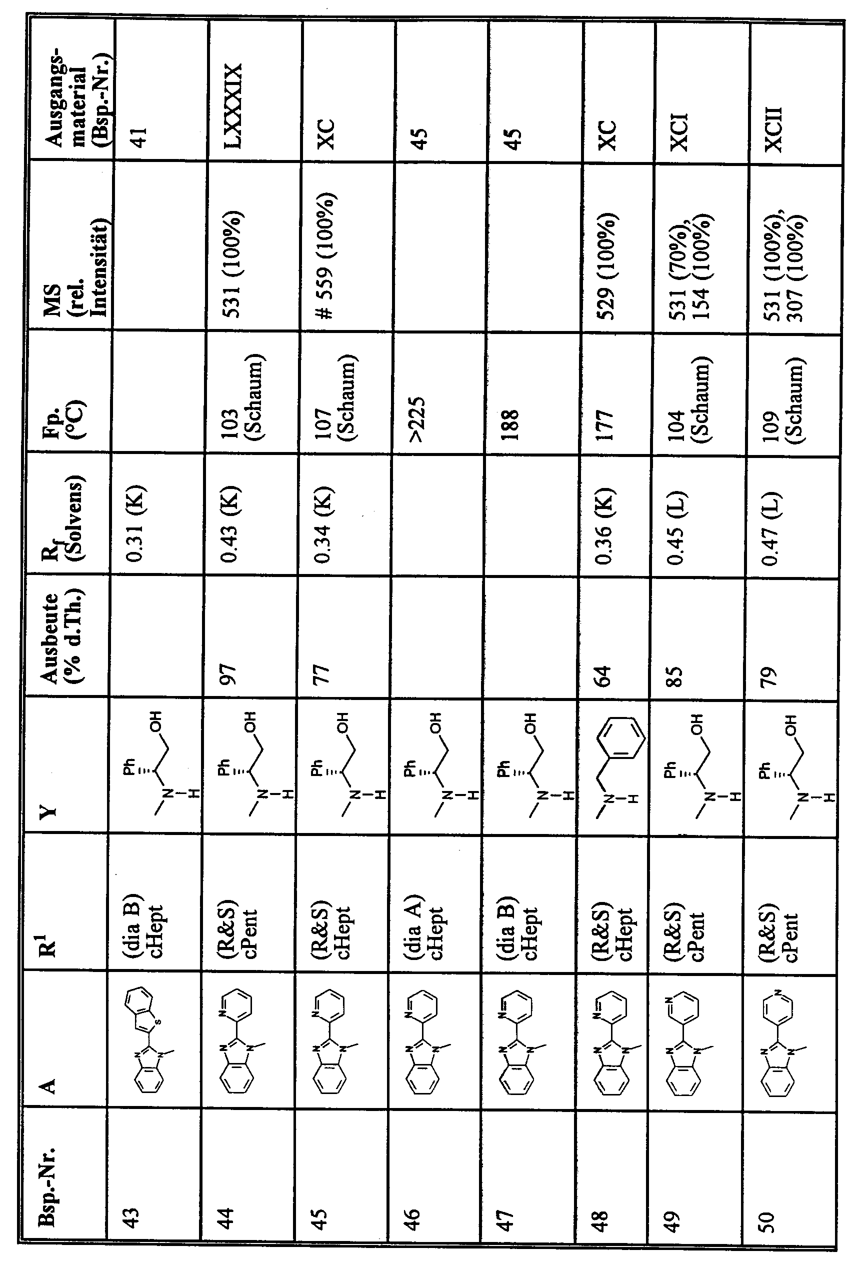

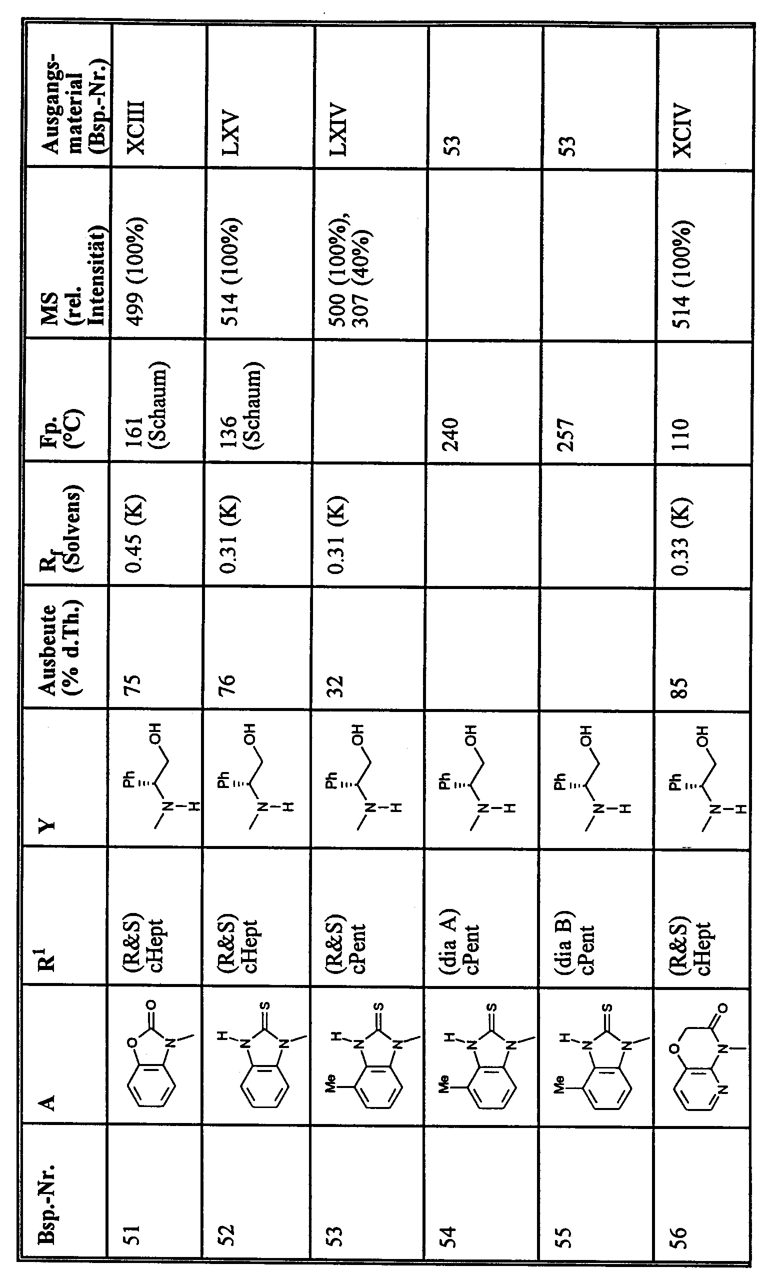

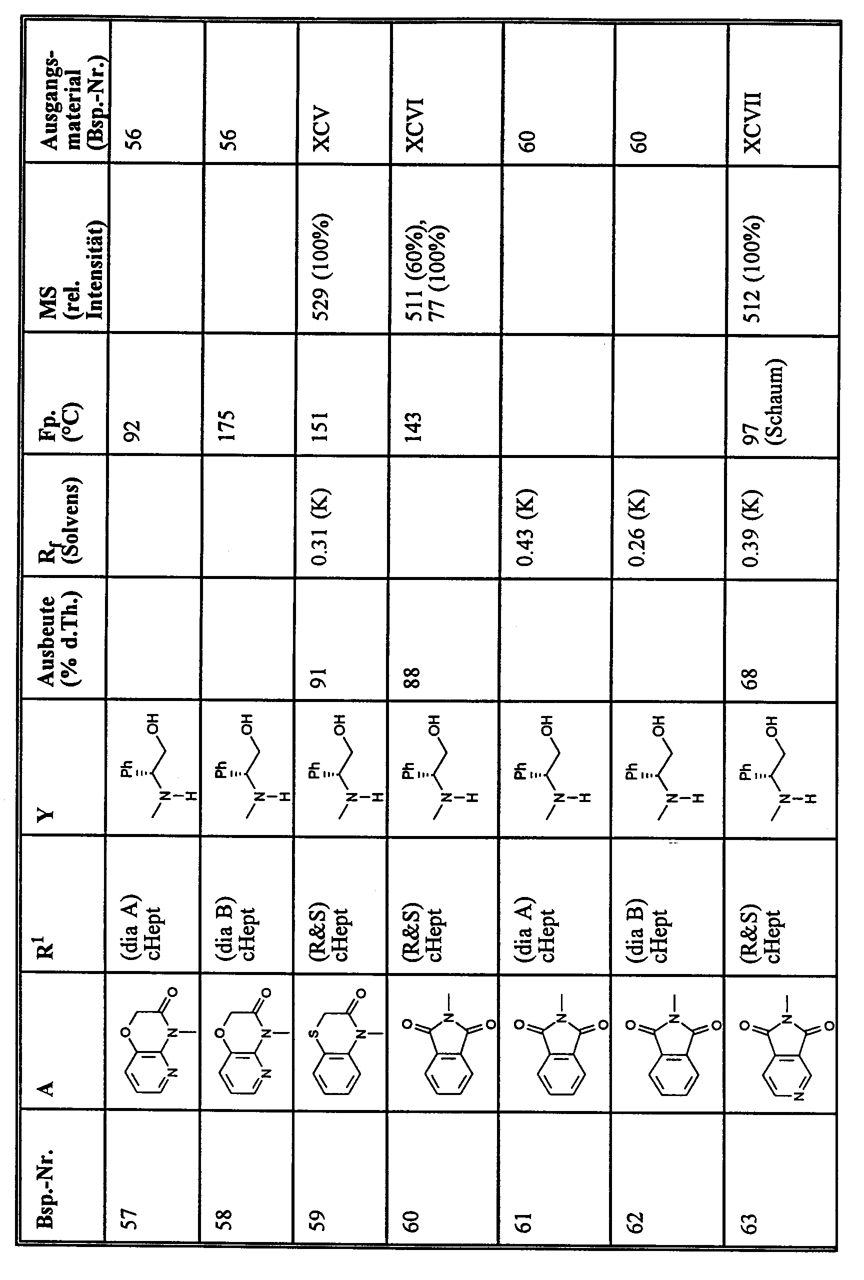

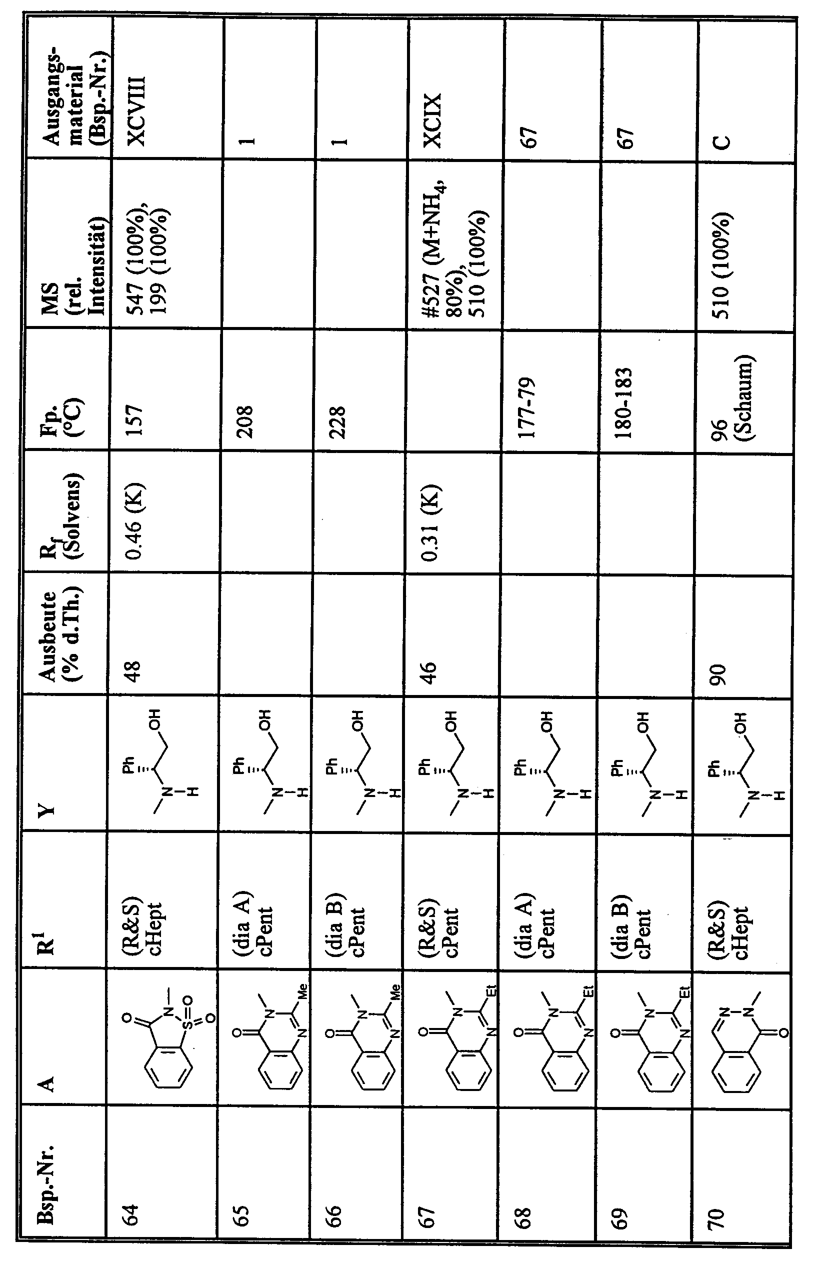

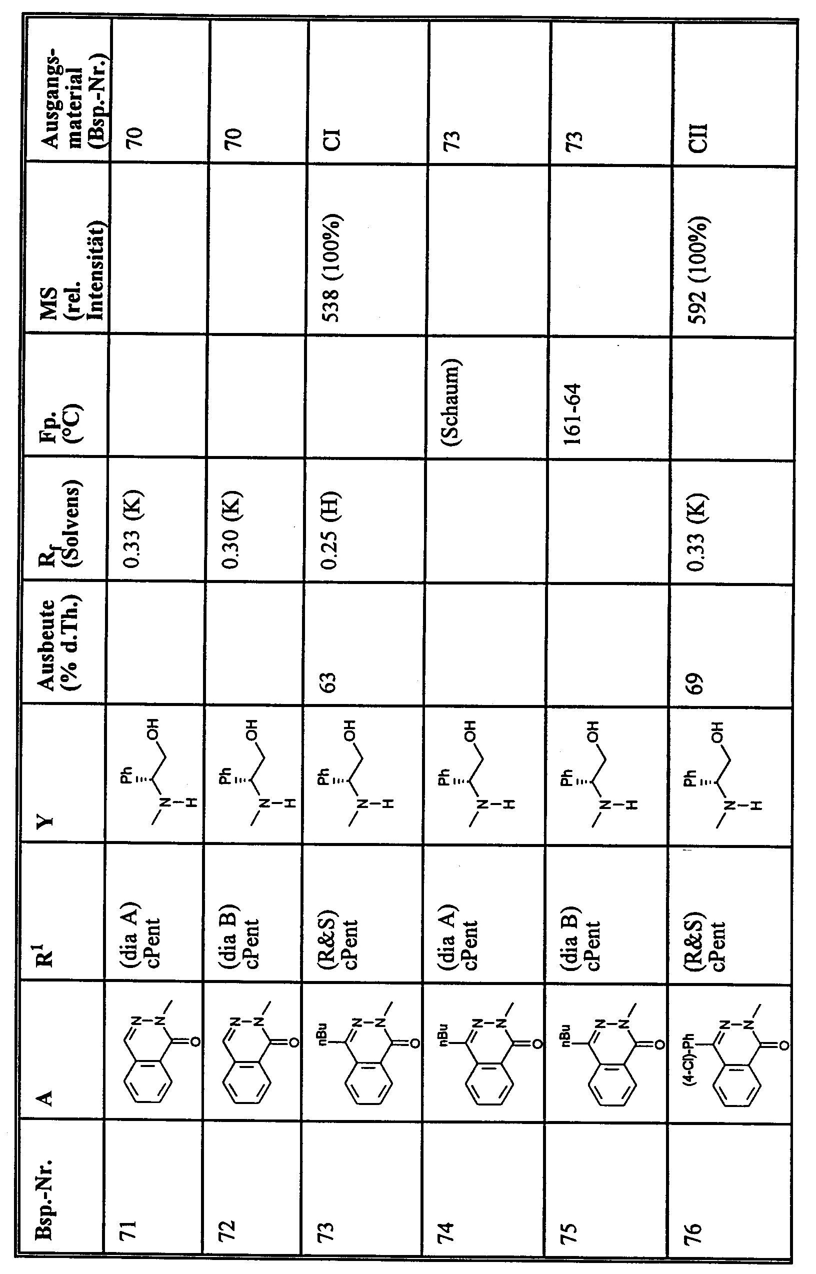

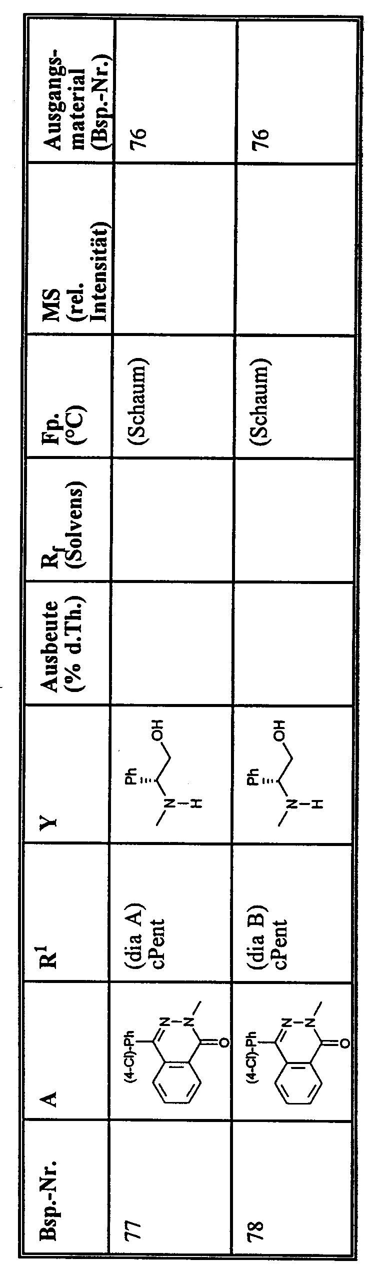

### Beispiel 79

### 2-(R/S)-Cycloheptyl-N-(2-hydroxy-1-(R)-phenylethyl)-2-[4-(2-methyl-(R/S)-sulfinyl-benzimidazol-1-ylmethyl)-phenyl]essigsäureamid

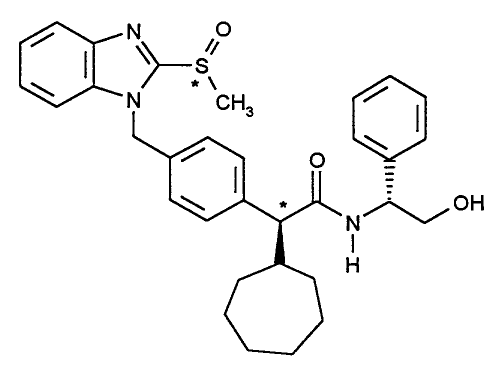

Man löst 0.30 g (0.568 mmol) der Verbindung aus Beispiel 6 in 10 ml CH₂Cl₂ und kühlt auf 0°C ab. Dazu gibt man langsam 0.178 g (5.68 mmol 55 %ig ) m-Chlorperbenzosäure. Es wird 30 Minuten bei 0°C und dann bei Raumtemperatur nachgerührt. Nach 1 Stunde gibt man etwas CH₂Cl₂ und ges. NaHCO₃-Lösung dazu und extrahiert. Die organische Phase wird über Natriumsulfat getrocknet, einrotiert und säulenchromatographisch gereinigt. (Kieselgel Merck 60 (0,040-0.063)):
Ausbeute: 33 mg (10.7 %);
Fp = 158°C;
R_{f} = 0.25 (CH₂Cl₂:Methanol, 100:5);
Masse (berechnet) für C₃₂H₃₇N₃O₃S = 543.74;
Massenspektrum (FAB, rel. Intensität) 444 (25%), 154 (80%), 55 (100%).

## Patentansprüche

1. Bicyclische Heterocyclen der allgemeinen Formel (I) 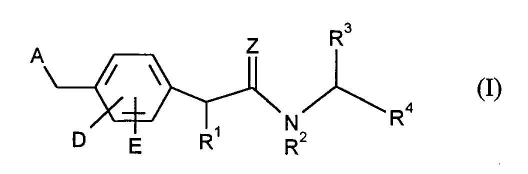 in welcher
A für einen Rest der Formel 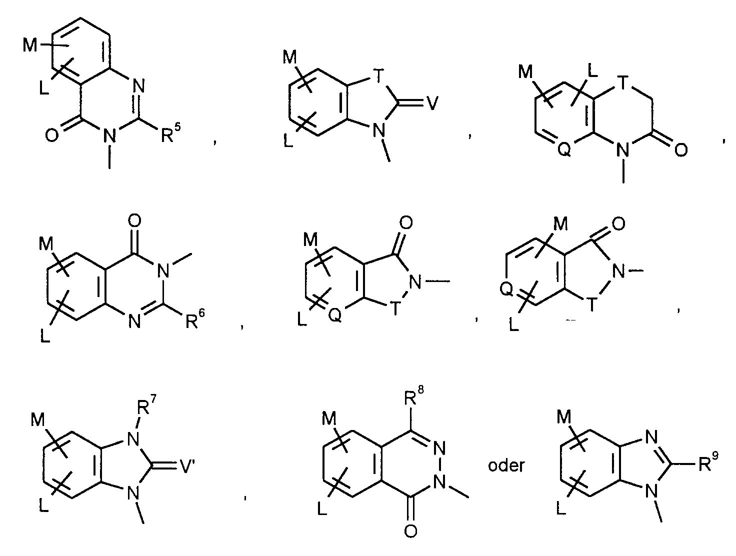 steht,
worin
L und M gleich oder verschieden sind und
Wasserstoff, Halogen, Trifluormethyl, Carboxyl, Cycloalkyl mit 3 bis 6 Kohlenstoffatomen, Hydroxy, Phenyl oder Wasserstoff Halogen, Trifluormethyl, Carboxyl, Cycloalkyl mit 3 bis 6 Kohlenstoffatomen, Hydroxy, Phenyl oder geradkettiges oder verzweigtes Alkyl, Alkoxycarbonyl oder Alkoxy mit jeweils bis zu 6 Kohlenstoffatomen bedeuten,
Q ein Stickstoffatom oder die -CH-Gruppe bedeutet,
T eine Gruppe der Formel -SO₂ oder -CO oder
ein Sauerstoff- oder Schwefelatom bedeutet,
V ein Sauerstoff- oder Schwefelatom bedeutet,
R⁵, R⁶, R⁷ und R⁸ gleich oder verschieden sind und
Wasserstoff geradkettiges oder verzweigtes Alkyl mit bis zu 6 Kohlenstoffatomen, Benzyl oder Phenyl bedeuten, die gegebenenfalls durch Halogen oder durch geradkettiges oder verzweigtes Alkyl mit bis zu 6 Kohlenstoffatomen substituiert sind,
R⁹ Trifluormethyl, Benzyl oder einen 5- bis 7-gliedrigen, gegebenenfalls benzokondensierten Heterocyclus mit bis zu 3 Heteratomen aus der Reihe S, N und/oder O bedeutet, der gegebenenfalls bis zu 3-fach gleich oder verschieden durch Halogen, Phenyl, Hydroxy oder durch geradkettiges oder verzweigtes Alkyl oder Alkoxy mit jeweils bis zu 4 Kohlenstoffatomen substituiert ist, oder
eine Gruppe der Formel -S(O)ₐ-R¹⁰ bedeutet,
worin
a eine Zahl 0, 1 oder 2 bedeutet,
R¹⁰ geradkettiges oder verzweigtes Alkyl oder Alkenyl mit jeweils bis zu 8 Kohlenstoffatomen bedeutet, die gegebenenfalls durch geradkettiges oder verzweigtes Acyl mit bis zu 6 Kohlenstoffatomen oder durch Aryl oder Aroyl mit jeweils bis zu 10 Kohlenstoffatomen substituiert sind, die ihrerseits bis zu 2-fach gleich oder verschieden durch Halogen, Trifluormethyl oder durch geradkettiges oder verzweigtes Acyl mit bis zu 5 Kohlenstoffatomen substituiert sein können, oder
Aryl mit 6 bis 10 Kohlenstoffatomen bedeutet, die gegebenenfalls durch Halogen, Hydroxy, Trifluormethyl oder geradkettiges oder verzweigtes Alkyl oder Alkoxy mit jeweils bis zu 5 Kohlenstoffatomen substituiert ist,
D und E gleich oder verschieden sind und
für Wasserstoff, Halogen, Trifluormethyl, Hydroxy, Carboxyl oder für geradkettiges oder verzweigtes Alkyl, Alkoxy oder Alkoxycarbonyl mit jeweils bis zu 6 Kohlenstoffatomen stehen,
Z für ein Sauerstoff- oder Schwefelatom steht,
R¹ für Cycloalkyl mit 3 bis 10 Kohlenstoffatomen oder
für geradkettiges oder verzweigtes Alkyl mit 1 bis 10 Kohlenstoffatomen steht, oder
für Phenyl steht, das gegebenenfalls bis zu 2-fach gleich oder verschieden durch Halogen, Nitro, Cyano, Hydroxy, geradkettiges oder verzweigtes Alkyl oder Alkoxy mit jeweils bis zu 4 Kohlenstoffatomen substituiert ist,
R² für Wasserstoff oder geradkettiges oder verzweigtes Alkyl mit bis zu 3 Kohlenstoffatomen steht,
R³ für Wasserstoff oder geradkettiges oder verzweigtes Alkyl mit bis zu 5 Kohlenstoffatomen steht, oder
für Cycloalkyl mit 3 bis 7 Kohlenstoffatomen steht, oder
für Phenyl oder für einen 5- bis 7-gliedrigen aromatischen Heterocyclus mit bis zu 3 Heteroatomen aus der Reihe S, N und/oder O steht, die gegebenenfalls bis zu 3-fach gleich oder verschieden durch Halogen, Nitro, Phenyl, Hydroxy oder durch geradkettiges oder verzweigtes Alkyl oder Alkoxy mit bis zu 6 Kohlenstoffatomen substituiert sind,
R⁴ für Wasserstoff oder für eine Gruppe der Formel -CH₂-OH oder CH₂O-CO-R¹¹ steht,
worin
R¹¹ Wasserstoff, geradkettiges oder verzweigtes Alkyl mit bis zu 8 Kohlenstoffatomen oder Phenyl bedeutet, das gegebenenfalls bis zu 3-fach gleich oder verschieden durch Halogen, Hydroxy, Cyano oder geradkettiges oder verzweigtes Alkyl oder Alkoxy mit jeweils bis zu 4 Kohlenstoffatomen substituiert ist,
und deren Salze.

2. Bicyclische Heterocyclen der Formel nach Anspruch 1, in welcher
A für einen Rest der Formel 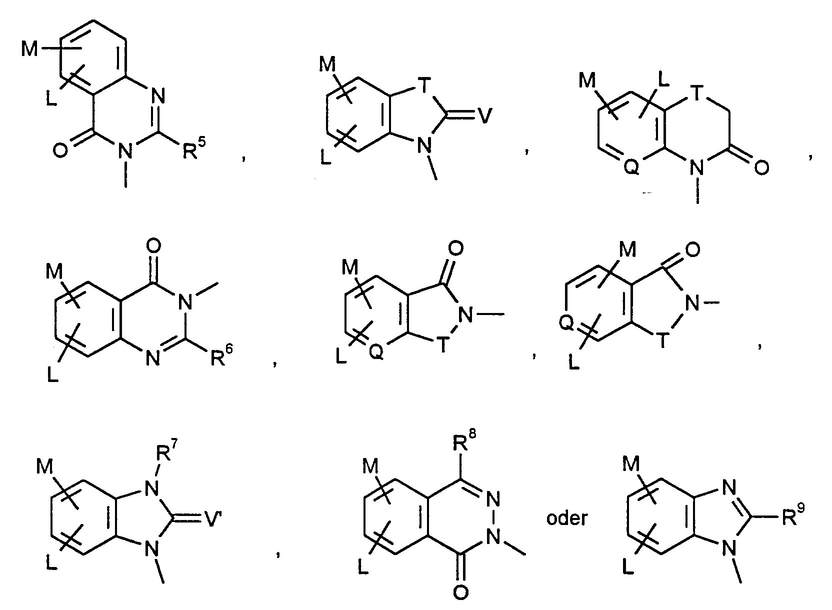 steht,
worin
L und M gleich oder verschieden sind und
Wasserstoff, Fluor, Chlor, Brom, Trifluormethyl, Cyclopropyl, Cyclobutyl, Cyclopentyl, Cyclohexyl, Hydroxy, Phenyl oder geradkettiges oder verzweigtes Alkyl, Alkoxycarbonyl oder Alkoxy mit jeweils bis zu 5 Kohlenstoffatomen bedeuten,
Q ein Stickstoffatom oder die -CH-Gruppe bedeutet,
T eine Gruppe der Formel -SO₂ oder -CO oder
ein Sauerstoff- oder Schwefelatom bedeutet,
V ein Sauerstoff- oder Schwefelatom bedeutet,
R⁵, R⁶, R⁷ und R⁸ gleich oder verschieden sind und
Wasserstoff, geradkettiges oder verzweigtes Alkyl mit bis zu 5 Kohlenstoffatomen, Benzyl oder Phenyl bedeuten, die gegebenenfalls durch Fluor, Chlor, Brom oder durch geradkettiges oder verzweigtes Alkyl mit bis zu 5 Kohlenstoffatomen substituiert sind,
R⁹ Trifluormethyl, Benzyl, Benzothienyl, Thienyl, Pyridyl, Chinolyl, Imidazolyl, Furyl, Pyrryl, Oxazolyl oder Thiazolyl bedeutet, die gegebenenfalls bis zu 3-fach gleich oder verschieden durch Fluor, Chlor, Brom, Phenyl, Hydroxy oder durch geradkettiges oder verzweigtes Alkyl oder Alkoxy mit jeweils bis zu 3 Kohlenstoffatomen substituiert sind, oder
eine Gruppe der Formel -S(O)ₐ-R¹⁰ bedeutet,
worin
a eine Zahl 0 oder 1 bedeutet,
R¹⁰ geradkettiges oder verzweigtes Alkyl oder Alkenyl mit jeweils bis zu 6 Kohlenstoffatomen bedeutet, die gegebenenfalls durch geradkettiges oder verzweigtes Acyl mit bis zu 5 Kohlenstoffatomen oder durch Phenyl, Benzoyl oder Naphthyl substituiert sind, die ihrerseits bis zu 2-fach gleich oder verschieden durch Fluor, Chlor, Brom, Trifluormethyl oder durch geradkettiges oder verzweigtes Acyl mit bis zu 4 Kohlenstoffatomen substituiert sein können, oder Naphthyl oder Phenyl bedeutet, die gegebenenfalls durch Fluor, Chlor, Brom, Hydroxy, Trifluormethyl oder geradkettiges oder verzweigtes Alkyl oder Alkoxy mit jeweils bis zu 4 Kohlenstoffatomen substituiert sind,
D und E gleich oder verschieden sind und
für Wasserstoff, Fluor, Chlor, Brom, Trifluormethyl, Hydroxy oder geradkettiges oder verzweigtes Alkyl oder Alkoxy mit jeweils bis zu 4 Kohlenstoffatomen stehen,
Z für ein Sauerstoff- oder Schwefelatom steht,
R¹ für Cyclobutyl, Cyclopentyl, Cyclohexyl, Cycloheptyl, Cylcooctyl oder für geradkettiges oder verzweigtes Alkyl mit bis zu 7 Kohlenstoffatomen steht, oder
für Phenyl steht, das gegebenenfalls durch Fluor, Chlor, Brom, Nitro, Cyano, Hydroxy oder geradkettiges oder verzweigtes Alkyl oder Alkoxy mit jeweils bis zu 3 Kohlenstoffatomen substituiert ist,
R² Wasserstoff oder Methyl bedeutet,
R³ für Wasserstoff oder geradkettiges oder verzweigtes Alkyl mit bis zu 4 Kohlenstoffatomen, Benzyl, Cyclopropyl, Cyclopentyl, Cyclohexyl oder für Phenyl, Pyridyl, Thienyl oder Furyl steht, die gegen benenfalls bis zu 2-fach gleich oder verschieden durch Fluor, Chlor, Brom, Phenyl, Nitro, Hydroxy oder durch geradkettiges oder verzweigtes Alkyl oder Alkoxy mit bis zu 4 Kohlenstoffatomen substituiert sind,
R⁴ für Wasserstoff oder für eine Gruppe der Formel -CH₂-OH oder -CH₂O-CO-R¹¹ steht,
worin
R¹¹ Wasserstoff, geradkettiges oder verzweigtes Alkyl mit bis zu 6 Kohlenstoffatomen oder Phenyl bedeutet, das gegebenenfalls bis zu 2-fach gleich oder verschieden durch Fluor, Chlor, Brom, Cyano, Hydroxy oder geradkettiges oder verzweigtes Alkyl oder Alkoxy mit jeweils bis zu 3 Kohlenstoffatomen substituiert ist,
und deren Salze.

3. Bicyclische Heterocyclen der Formel nach Anspruch 1, in welcher
A für einen Rest der Formel 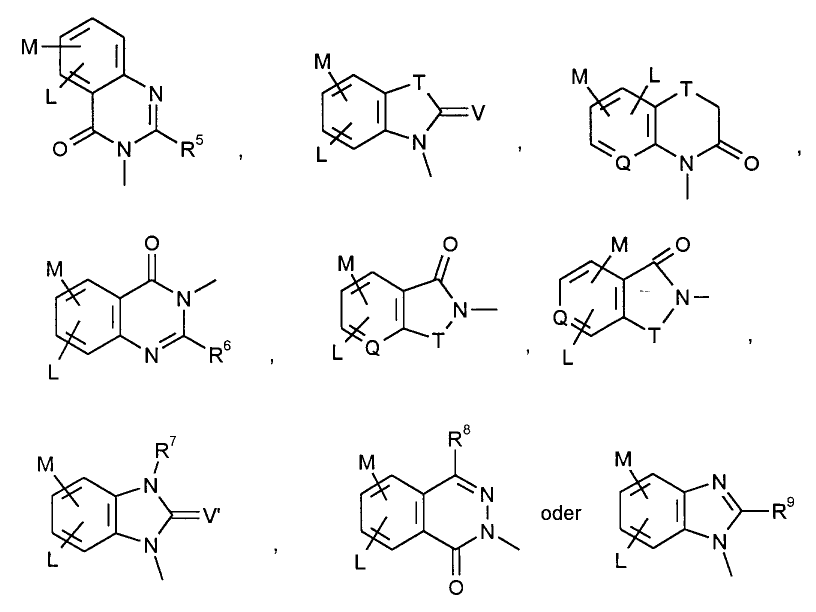 steht,
worin
L und M gleich oder verschieden sind und
Wasserstoff, Fluor, Chlor, Brom, Hydroxy, Phenyl oder geradkettiges oder verzweigtes Alkyl oder Alkoxy mit jeweils bis zu 4 Kohlenstoffatomen bedeuten,
Q ein Stickstoffatom oder die -CH-Gruppe bedeutet,
T eine Gruppe der Formel -SO₂ oder -CO oder
ein Sauerstoff- oder Schwefelatom bedeutet,
V ein Sauerstoff- oder Schwefelatom bedeutet,
R⁵, R⁶, R⁷ und R⁸ gleich oder verschieden sind und
Wasserstoff, geradkettiges oder verzweigtes Alkyl mit bis zu 4 Kohlenstoffatomen, Benzyl oder Phenyl bedeuten, die gegebenenfalls durch Fluor, Chlor, Brom oder durch geradkettiges oder verzweigtes Alkyl mit bis zu 4 Kohlenstoffatomen substituiert sind,
R⁹ Trifluormethyl, Benzyl, Benzothienyl, Thienyl, Pyridyl, Imidazolyl, Furyl oder Thiazolyl bedeutet, die gegebenenfalls bis zu 3-fach gleich oder verschieden durch Fluor, Chlor, Brom, Phenyl, Hydroxy oder durch geradkettiges oder verzweigtes Alkyl oder Alkoxy mit jeweils bis zu 3 Kohlenstoffatomen substituiert sind, oder
eine Gruppe der Formel -S(O)ₐ-R¹⁰ bedeutet,
worin
a eine Zahl 0 oder 1 bedeutet,
R¹⁰ geradkettiges oder verzweigtes Alkyl oder Alkenyl mit jeweils bis zu 5 Kohlenstoffatomen bedeutet, die gegebenenfalls durch geradkettiges oder verzweigtes Acyl mit bis zu 4 Kohlenstoffatomen oder durch Phenyl, Benzoyl oder Naphthyl substituiert sind, die ihrerseits bis zu 2-fach gleich oder verschieden durch Fluor, Chlor, Brom, Trifluormethyl oder durch geradkettiges oder verzweigtes Acyl mit bis zu 3 Kohlenstoffatomen substituiert sein können, oder
Naphthyl oder Phenyl bedeutet, die gegebenenfalls durch Fluor, Chlor, Brom, Hydroxy, Trifluormethyl oder geradkettiges oder verzweigtes Alkyl oder Alkoxy mit jeweils bis zu 3 Kohlenstoffatomen substituiert sind,
D und E gleich oder verschieden sind und
für Wasserstoff, Fluor, Chlor, Brom oder Trifluormethyl stehen,
Z für Sauerstoff steht,
R¹ für Cyclopentyl, Cyclohexyl, Cycloheptyl, Cylcooctyl oder für geradkettiges oder verzweigtes Alkyl mit bis zu 6 Kohlenstoffatomen steht,
R² für Wasserstoff oder Methyl steht,
R³ für Phenyl steht und
R⁴ für die Gruppe -CH₂-OH steht,
und deren Salze.

4. Bicyclische Heterocyclen nach Anspruch 1 bis 3 zur therapeutischen Anwendung.

5. Verfahren zur Herstellung von bicyclischen Heterocyclen nach Anspruch 1 bis 3, dadurch gekennzeichnet, daß man Säuren der allgemeinen Formel (II) 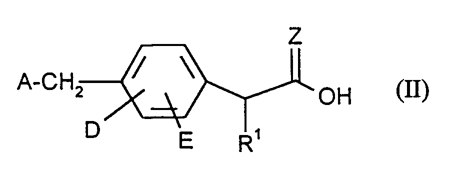 in welcher
A, D, E, Z und R¹ die angegebene Bedeutung haben,
mit Verbindungen der allgemeinen Formel (III) 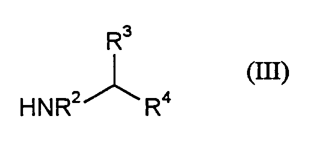 in welcher
R², R³ und R⁴ die angegebene Bedeutung haben,
in inerten Lösemitteln und in Anwesenheit von Basen und/oder Hilfsstoffen umsetzt.

6. Arzneimittel enthaltend bicyclische Heterocyclen nach Anspruch 1 bis 3.

7. Arzneimittel nach Anspruch 6 zur Behandlung von Atherosklerose.

8. Verfahren zur Herstellung von Arzneimitteln nach Anspruch 6 und 7, dadurch gekennzeichnet, daß man die bicyclischen Heterocyclen gegebenenfalls mit üblichen Hilfs- und Trägerstoffen in eine geeignete Applikationsform überführt.

9. Verwendung von bicyclischen Heterocyclen nach Anspruch 1 bis 3 zur Herstellung von Arzneimitteln.

10. Verwendung nach Anspruch 9 zur Herstellung von antiatherosklerotischen Arzneimitteln.
